# EUROPÄISCHE PATENTSCHRIFT

(11) **EP 4 125 750 B1**
(45) Veröffentlichungstag und Bekanntmachung des Hinweises auf die Patenterteilung: **07.05.2025**
(21) Anmeldenummer: 21715575.3
(22) Anmeldetag: 26.03.2021
(51) Int. Cl.: A61F 9/008

(54) **VORRICHTUNG ZUR REFRAKTIVEN CHIRURGIE, INSBESONDERE ZUR KERATOPLASTIE**
EQUIPMENT FOR REFRACTIVE SURGERY, PARTICULARLY FOR KERATOPLASTY
ÉQUIPEMENT DE CHIRURGIE RÉFRACTIVE, EN PARTICULIER POUR KÉRATOPLASTIE

(30) Priorität: 01.04.2020 DE 102020204261
(43) Veröffentlichungstag der Anmeldung: 08.02.2023
(73) Patentinhaber: Carl Zeiss Meditec AG, 07745 Jena (DE)
(72) Erfinder: BISCHOFF, Mark, 07745 Jena (DE); POMRAENKE, Robert, 07745 Jena (DE)
(74) Vertreter: Rößner, Ulrike
(86) Internationale Anmeldenummer: PCT/EP2021/058022
(87) Internationale Veröffentlichungsnummer: WO 2021/198106

(56) Entgegenhaltungen:
- EP-A2- 1 109 007
- EP-B1- 2 892 464
- WO-A1-2008/131888
- WO-A1-2011/069516
- WO-A1-2020/212199
- WO-A2-01/89373
- WO-A2-2006/128038
- CN-A- 1 043 257
- DE-A1- 102007 019 815
- DE-A1- 102007 019 815
- RU-C1- 2 652 753
- US-A- 5 789 240
- US-A1- 2003 014 042
- US-A1- 2009 247 999
- US-A1- 2014 264 980
- US-A1- 2018 206 482
- HOMOLKA P ET AL: "DAS EXCIMER LASER CORNEAL SHAPING-(ELCS-)SYSTEM: HOCHPRAEZISE HERSTELLUNG VON HORNHAUT- (CORNEA-)TRANSPLANTATEN MITTELS EXCIMER LASER-ABLATION", ELEKTROTECHNIK UND INFORMATIONSTECHNIK, SPRINGER VERLAG, WIEN, AT, vol. 117, no. 4, 1 January 2000 (2000-01-01), pages 265 - 268, XP001107554, ISSN: 0932-383X

## Beschreibung

Die vorliegende Erfindung betrifft Vorrichtungen der refraktive Chirurgie, insbesondere zur Keratoplastie, beispielsweise eine nahtlose lamelläre instrastromale Keratoplastie (sutureless Intrastromal Anterior Lamellar Keratoplasty = sIALK).

Vorrichtungen und Verfahren der refraktiven Chirurgie, insbesondere zur Keratoplastie, wie zum Beispiel beschrieben in der DE 10 2007 019 815 A1 und WO 2008/131888 A1, gehen bislang davon aus, dass aus dem Empfängerauge im Zuge der Behandlung immer ein Gewebestück entnommen wird. Bisher ist hierfür das Verfahren der sIALK so beschrieben, dass stets eine Resektion erfolgen muss, weil erst dadurch eine Vertiefung im stromalen Bett (Vakanz) entsteht, die eine lagerichtige Implantation eines Implantats oder Transplantats erleichtert. Eine Vakanz hat inhärente Nachteile, nämlich die mit der Erzeugung verbundene chirurgische Belastung und den Gewebeverlust. Oft überwiegen aber die Vorteile: die Gewinnung von Biopsie-Material und die vereinfachte Positionierung des Implantats.

In einer bestimmten Ausgestaltung erfolgt neben einer Verbesserung des Brillenvisus (CDVA) auch eine Verbesserung des unkorrigiertes Visus (UDVA). Es wird also gleichzeitig eine Brillenkorrektur durchgeführt. Dieser Gedanke ist ebenfalls in Teilen bereits in DE 10 2007 019 815 A1 bzw. WO 2008/131888 A1 formuliert.

Die Formgebung kann im Rahmen der Herstellung des Implantats, insbesondere eines Gewebe-Implantats, auf unterschiedliche Weise erfolgen. In US 2009/0247999 A1 wird hierzu ein System offenbart, mit dem das Implantat im Auge des Patienten nachbearbeitet wird. Ein weiteres System zur Erzeugung von Implantaten ist aus US 2014/264980 A1 bekannt.

Eine mögliche Vorgehensweise besteht darin, einem Implantat-Rohling mittels Laserbearbeitung die Zielgeometrie aufzuprägen. Dies ist insbesondere für die refraktive Chirurgie ein interessantes Vorgehen, weil die dort üblichen refraktiven Lasersysteme aufwandgering für solche Verfahren angepasst werden könnten. Somit ließe sich die patientenspezifische Anpassung des Implantats durch den medizinischen Anwender in der Klinik durchführen und müsste nicht in einer Gewebebank oder anderswo erfolgen. Ein solcher Ansatz hätte also logistische Vorteile und würde auch einige Risiken für die Patienten verringern.

Bei einfacher Übertragung der Vorgehensweise einer PRK- oder LASIK-Behandlung auf das Problem der Implantat-Herstellung lässt sich jedoch die erforderliche Bearbeitungsgenauigkeit nicht zuverlässig erzielen. Denn im Unterschied zur üblichen Verwendung bei der Formung stromalen Hornhautgewebes zur subtraktiven Korrektur eines Sehfehlers, bei der pro Dioptrie Korrektur etwa bis zu 13 µm dicke Gewebeschichten entfernt werden und somit selten mehr als 100 µm maximaler Abtrag erfolgt, ist ein Abtrag von mehr als 100 µm Schichtdicke bei der Implantat-Herstellung nicht ungewöhnlich. Bei einem so großen Abtrag letztlich eine absolute Genauigkeit im Mikrometer-Bereich zu erzielen ist schwieriger als die hohe relative Genauigkeitsanforderung einer PRK-Korrektur zu erzielen, zumal die Eigenschaften des Implantat-Materials größeren Schwankungen unterworfen sein können als dies beim stromalen Gewebe während einer PRK- oder LASIK-Behandlung der Fall ist.

Neben der Formgebung des Implantats innerhalb der Korrekturzone des Gewebes, das ein natürliches Spender-Hornhautgewebe oder ein künstliches Gewebe mit identischen Eigenschaften wie ein natürliches Hornhautgewebe sein kann, ist bei der Geometrie seines Randes eine besonders hohe Genauigkeit erforderlich. In dieser als Rand bezeichneten Peripherie des Implantats ist oft eine genaue Einpassung in eine vorhandene Vakanz im Gewebe notwendig. (Hierbei ist eine Genauigkeit von wenigstens 10 µm, möglichst aber von 5 µm oder weniger zu erreichen. Im Idealfall kann eine Anpassung auf 1 µm genau erfolgen.) Ein Aspekt dieses Problems besteht darin, dass der Chirurg den Rand des Implantats während des gesamten Prozesses nicht beschädigen darf, beispielsweise dann, wenn er das zu transplantierende Gewebe vom umgebenden Gewebe des Spenderauges trennt, oder das Material von nicht benötigten Teilen. Diese Prozessschritte erfordern naturgemäß manuelle mikrochirurgische Arbeit mit Chirurgie-Instrumenten.

Aufgabe der Erfindung ist es, Vorrichtungen zur Herstellung und Implantation einer Lamelle eines Gewebes bzw. Materials zum Zweck der Korrektur einer Hornhautgeometrie mit höchster und damit gegenüber dem Stand der Technik verbesserter Präzision zu beschreiben. Ziel der Korrektur ist insbesondere die Wiederherstellung einer normalen Hornhautgeometrie mit gegenüber dem Stand der Technik verbesserter optischer Funktion der Hornhaut.

Die Erfindung gliedert sich in verschiedene Maßnahmen bzw. Merkmale, die alle dem Zweck der Verbesserung der o.g. Vorrichtungen der refraktive Chirurgie, insbesondere zur Keratoplastie, dienen und letztendlich zur Erreichung eines gemeinsamen Ziels eingesetzt werden: der Herstellung und Implantation eines Gewebes bzw. Materials zum Zweck der Korrektur einer Hornhautgeometrie mit gegenüber dem Stand der Technik verbesserter Präzision.

Mit der hier durch die beschriebenen erfindungsgemäßen Vorrichtungen angestrebten optimierten Zielgeometrie soll die optische Funktion der Hornhaut (auch Cornea genannt) verbessert werden und gleichzeitig eine annähernd normale Hornhautgeometrie hergestellt bzw. wiederhergestellt werden.

Die Maßnahmen bzw. Merkmale sollen zu einer verbesserten Form des Implantats (zur Vermeidung von Hohlräumen) und zu einer verbesserten Bearbeitungsgenauigkeit führen. So ist es insbesondere Aufgabe der Erfindung, die Peripherie des (natürlichen oder künstlichen) Gewebes bzw. Materials optimal für die manuellen Verfahrensschritte vorzubereiten, bzw. kritische Verfahrensschritte durch Laserbearbeitung zu erleichtern oder, wo möglich, auch zu ersetzen, sowie Maßnahmen zum Zweck der Verbesserung der Genauigkeit der Laserbearbeitung und der Verringerung der Belastung bzw. Schädigung des Implantats zu beschreiben.

Die Erfindung ist in dem unabhängigen Anspruch definiert. Die abhängigen Ansprüche betreffen bevorzugte Weiterbildungen.

Bevor diese Gruppen im Detail beschrieben werden, sollen jedoch einige Begriffe geklärt werden:
- "Implantat" ist ein Gewebe, hier insbesondere ein (ggf. modifiziertes) Hornhautgewebe eines Spender-Auges oder ein künstliches Gewebe bzw. Material nicht-menschlichen Ursprungs mit identischen Eigenschaften.
- "Transplantat" ist ein Gewebe menschlichen Ursprungs. (Die Unterscheidung anhand enthaltener lebender Zellen mag regulatorisch relevant sein, soll hier aber vernachlässigt werden.)
- "Implantation" meint das Einsetzen sowohl eines Implantats als auch eines Transplantats.
- "Blank" meint einen Rohling. Dieser Rohling kann ein Implantat oder Transplantat sein, welches im Allgemeinen Fall eine dreidimensional gekrümmte, runde Grundform mit einem Durchmesser von ca. 5-9 mm und eine Dicke zwischen 10 µm und 400 µm, maximal 500µm, hat. Das Dickenprofil dieser Kugelschale bzw. dieses kugelschalenähnlichen Gebildes mit einem Krümmungsradius zwischen 5 mm und Unendlich ist noch nicht an das Empfängerauge angepasst. Aus dem Blank entsteht durch Bearbeitung eine Lamelle.
- "Lamelle" meint ein Implantat oder Transplantat, welches aus einem Blank hergestellt wird und ein Dickenprofil erhält, welches an das Empfängerauge speziell angepasst ist. Die Lamelle ist folglich das finale Produkt, bereit zum Einführen in eine dafür vorbereitete Schnittfläche in der Hornhaut eines Empfängerauges bzw. eine durch diese Schnittfläche definierte Vakanz (Struktur).
- "Vakanz" bezeichnet die durch die Resektion (also Entfernung eines Hornhaut-Volumens) entstehende Struktur im stromalen Bett. Man kann die Vakanz auch als Resektions-Kavität bezeichnen, obwohl sie praktisch nie wirklich als Hohlraum in der Hornhaut existiert, sondern sich die darüber befindliche Lamelle stets weitgehend an das stromale Bett anlegt und ein zunächst entstandener Hohlraum schnell mit Gewebsflüssigkeit gefüllt wird.

Die Aufgabe wird gelöst durch eine erfindungsgemäße Planungseinrichtung zum Erzeugen von Steuerdaten für ein Behandlungssystem zur refraktiven Chirurgie, insbesondere zur Keratoplastie, das eine erste Lasereinrichtung und mindestens eine Charakterisierungseinrichtung umfasst, wobei mittels der Steuerdaten die erste Lasereinrichtung, vorzugsweise eine Femtosekunden-Lasereinrichtung, zur Erzeugung mindestens einer Schnittfläche in einer Hornhaut eines Auges steuerbar ist. Aufgrund der Aufgaben, die diese erste Lasereinrichtung zu erfüllen hat, ist es notwendig, dass ein von ihr ausgesendeter Bearbeitungslaserstrahl in ein Augengewebe, insbesondere in die Hornhaut eines Auges eindringen und innerhalb des Gewebes eine Schnittfläche erzeugen kann. Hierfür eignen sich üblicherweise Lasereinrichtungen, deren Bearbeitungslaserstrahl an einem Fokus im Gewebe eine Photodisruption des Gewebes erzeugen kann.

Die Planungseinrichtung weist weiterhin auf:
- eine Schnittstelle zum Zuführen von ersten Messdaten über Parameter der Hornhaut der Charakterisierungseinrichtung, vorzugsweise einer OCT (optische Kohärenz-Tomographie)-Einrichtung,
- eine Schnittstelle zum Zuführen von zweiten Messdaten oder Modelldaten einer Lamelle, die in die Hornhaut nach Erzeugung der Schnittfläche einführbar ist,
- eine Schnittstelle zum Abführen von Steuerdaten zur ersten Lasereinrichtung, und
- Berechnungsmittel zum Festlegen der mindestens einen Schnittfläche in der Hornhaut unter Verwendung der ersten Messdaten und der zweiten Messdaten oder Modelldaten, wobei die Berechnungsmittel einen Steuerdatensatz zur Ansteuerung der ersten Lasereinrichtung erzeugen, wobei durch die ersten Lasereinrichtung mit den Steuerdatensatz die mindestens eine Schnittfläche erzeugbar ist.

Die zu erzeugende mindestens eine Schnittfläche in der Hornhaut eines Auges, des Empfängerauges, kann dabei einfacher Natur sein und wirklich nur eine i.d.R. gekrümmte Fläche aufweisen, in die die Lamelle "eingeschoben" werden kann. Es kann aber auch - durch mehrere Schnittflächen - ein Volumen umschrieben sein, dass entnommen werden kann, um später in die dadurch entstehende Vakanz die Lamelle einzuführen.

Die Planungseinrichtung ist weiterhin eingerichtet, Steuerdaten für eine zweite Lasereinrichtung des Behandlungssystems, vorzugsweise eine Excimer-Lasereinrichtung, zu erzeugen zur Bearbeitung eines Blanks zur patientenspezifisch geformten Lamelle und weist eine Schnittstelle zum Abführen von Steuerdaten zur zweiten Lasereinrichtung auf, wobei erste Lasereinrichtung, zweite Lasereinrichtung und Charakterisierungseinrichtung jeweils ein Gerätekoordinatensystem aufweisen, und sie mittels Registrierung der Gerätekoordinatensysteme zueinander gekoppelt bzw. koppelbar und die zugeführten zweiten Messdaten oder Modelldaten der Lamelle zu diesen Gerätekoordinatensystemen registrierbar sind. Aufgrund der Aufgaben, die diese zweite Lasereinrichtung zu erfüllen hat, ist es notwendig, dass ein von ihr ausgesendeter Bearbeitungslaserstrahl ein Augengewebe, insbesondere die Hornhaut eines Auges von seiner Oberfläche ins Gewebe hinein abtragen kann. Obgleich natürlich auch hier mit einem eine Photodisruption im Gewebe erzeugenden Bearbeitungslaserstrahl gearbeitet werden kann, eignen sich hierfür insbesondere Lasereinrichtungen, deren Bearbeitungslaserstrahl das Gewebe mittels Ablation bearbeiten kann.

Die Bearbeitung des Blanks, zum Beispiel mit einer Excimer-Lasereinrichtung, erfolgt ebenfalls so, dass das Bearbeitungsprofil der Lasereinrichtung (Fluence bzw. Laserschussverteilung als Funktion des Ortes) exakt auf dem Blank "platziert" wird, dies erfordert im Normalfall eine Zentrierung mit einer Genauigkeit von etwa 100 µm. Dadurch wird das Blank zur patientenspezifischen Lamelle, deren Implantation die beabsichtigte Regularisierung der Pachymetrie und ggf. Refraktionsanpassung bewirkt.

Der ganzheitlichen Planung der Operation kommt eine wichtige Bedeutung zu. Mittels der Planungseinrichtung wird hierzu ein Planungsverfahren ausgeführt, dass in einer Planungs-Software in der Planungseinrichtung kodiert ist. Dafür werden als Input für die Planungs-Software die gemessenen Hornhaut-Tomografie-Daten und ggf. weitere Biometrie-Parameter (z.B. Krümmungsradius, Refraktion) des zu behandelnden Auges verwendet. Die Planungseinrichtung, in der die Planungs-Software kodiert ist, kann hierfür integrativer Bestandteil des Behandlungssystems sein oder aber parallel oder alternativ auf einem oder mehreren räumlich vom Behandlungssystem separierten Computern verteilt lokalisiert sein.

Dem Anwender wird dabei die ortsaufgelöste Pachymetrie des Patienten angezeigt und mindestens eine weitere Lagemarkierung (z.B. Mittelpunkt der photopischen Pupille bzw. corneal sight center, Vertex-Position, Limbus). Zudem verfügt die Software über die Information der typischen Pachy-Map eines gesunden Auges. Durch lagerichtige Differenzbildung der bestehenden Pachy-Map mit einer typischen Pachy-Map ermittelt die Software automatisch eine Differenz-Map. Der Anwender entscheidet, ob das Verfahren mit oder ohne Vakanz durchgeführt werden soll, definiert die Behandlungszone (in der Regel rund), indem er beispielsweise Lamellenzentrum und Lamellendurchmesser definiert, sowie weitere Geometrieparameter, wie beispielsweise Durchmesser und Randdicke der Vakanz, Randdicke der Lamelle, Tiefe des Pocket-Schnitts unter der Cornea-Oberfläche. Die Software kann einige oder alle diese Parameter auch automatisch vorschlagen bzw. festlegen. Dann erzeugt die Software sowohl Steuerdaten für die erste Lasereinrichtung, bevorzugt also einer Femtosekunden-Lasereinrichtung als auch für die zweite Lasereinrichtung, bevorzugt die Excimer-Lasereinrichtung.

Je nach Zustand des Blanks, aus dem die patientenspezifischen Lamelle gearbeitet werden soll, ist es ggf. von großem Vorteil, wenn die erfindungsgemäße Planungseinrichtung weiterhin eingerichtet ist, Steuerdaten für die erste Lasereinrichtung oder eine weitere Lasereinrichtung, ebenfalls vorzugsweise eine Femtosekunden-Lasereinrichtung, dessen Gerätekoordinatensystem ebenfalls zu den vorgenannten Gerätekoordinatensystemen mittels Registrierung gekoppelt ist, zu generieren zur Erzeugung bzw. Vorbearbeitung des Blanks, wobei das Blank aus einer natürlichen Spender-Hornhaut oder aus einem künstlichem Gewebe erzeugbar bzw. darin vorbearbeitbar ist durch eine Erzeugung einer oder mehrerer Schnittflächen in der Spender-Hornhaut oder dem künstlichen Gewebe mittels der ersten Lasereinrichtung oder der weiteren Lasereinrichtung.

Diese Steuerdaten können und sollten dafür eine Registrierinformation beinhalten, insbesondere:
1. Die Steuerdaten für beispielsweise eine Femtosekunden-Lasereinrichtung als vorbearbeitende weitere Lasereinrichtung zur Erzeugung eines Blanks in einem Ausgangsmaterial, ggf. in der Hornhaut eines Spenderauges oder einem künstlichen Gewebe. Falls das Ausgangsmaterial bereits in einer geeigneten Konfiguration vorliegt, kann dieser Schritt durch die Dateneinspeisung über die Geometrie des Materials ersetzt werden.
2. Die Steuerdaten für beispielsweise einen Excimer-Lasereinrichtung als nachbearbeitende zweite Lasereinrichtung zur Bearbeitung des Blanks zur Lamelle.
3. Die Steuerdaten zur Erzeugung mindestens einer Schnittfläche, also beispielsweise eines Pocket-Schnitts bzw. einer Vakanz im Empfängerauge zur Aufnahme der Lamelle, für beispielsweise eine Femtosekunden-Lasereinrichtung als erste Lasereinrichtung.

Eine bevorzugten Ausführungsform der erfindungsgemäßen Planungseinrichtung ist eingerichtet, Steuerdaten für ein Temperaturregime für die Einhaltung einer Temperatur kleiner einer Maximaltemperatur für die Bearbeitung der des Blanks zur Lamelle mit der zweiten Lasereinrichtung zu erzeugen. Eine Bearbeitung des Blanks zur Lamelle mittels Ablation durch diese zweite Lasereinrichtung führt i.d.R. zu einer Erwärmung des zu bearbeitenden Gewebes aufgrund des hohen Energieeintrags. Um eine präzise Strukturierung gewährleisten zu können, sollte die Bearbeitungstemperatur des Gewebes jedoch nicht schwanken, sondern besser auf einer niedrigen Temperatur konstant gehalten werden.

In der Erfindung wird das Blank vor und/oder während der Bearbeitung mit der zweiten Lasereinrichtung, zumeist einer Excimer-Lasereinrichtung, aktiv gekühlt. In einer speziellen Variante der Erfindung erfolgt die Kühlung unter 10 °C. In einer anderen Variante der Erfindung erfolgt die Kühlung unter den Gefrierpunkt des Blanks. Die Abprodukte der Bearbeitung werden mittels kontrollierter Luftströmung aktiv entfernt. In einer Variante der Erfindung wird der Luftstrom hinsichtlich Temperatur und/oder Luftfeuchtigkeit aktiv gesteuert. In einer Variante der Erfindung erfolgt die Kühlung auf den Taupunkt der Luft im Luftstrom. In einer anderen Variante der Erfindung wird statt Luft ein technisches Gas (z.B. Stickstoff) verwendet. In einer weiteren Variante der Erfindung wird die Bearbeitung kontinuierlich oder zyklisch überwacht. Überwachte Parameter sind beispielsweise Oberflächentemperatur, Abtragsvolumen, Materialdicke, Oberflächentopografie, axiale und laterale Position des Blanks. In einer Ausgestaltung der Erfindung werden die Überwachungsparameter zur Regelung des Abtragsprozesses verwendet.

Eine wichtige Überlegung bei der Planung (und späteren Ausführung) der Erzeugung einer patientenspezifischen Lamelle mit höchster Präzision betrifft das Vorsehen verschiedener Zonen des Blanks und der späteren Lamelle.

Die Planungseinrichtung ist eingerichtet, eine im Wesentlichen ringförmige Übergangszone am Rande der Lamelle zu bestimmen, innerhalb der die Randdicke allmählich in ein patientenspezifisches Dickenprofil übergeht, und weiterhin Steuerdaten so erzeugt werden, dass keine Bearbeitung des Randes der Lamelle durch die zweite Lasereinrichtung erfolgt, wobei die zweite Lasereinrichtung zur Bearbeitung des Blanks zur patientenspezifisch geformten Lamelle einen Halter aufweist, auf den das Blank während der Bearbeitung durch die zweite Lasereinrichtung fixierbar ist.

Bei Nutzung eines Halters für die Bearbeitung des Blanks mittels der zweiten Lasereinrichtung ist die Planungseinrichtung eingerichtet Steuerdaten für eine aktive Kühlung des Halters zu erzeugen.

Sollte das Blank nicht schon die geeigneten Maße für die Bearbeitung mittels der zweiten Lasereinrichtung aufweisen, so ist in einer ersten vorteilhaften Variante die Planungseinrichtung eingerichtet, Schnittflächen derart in der Spender-Hornhaut oder dem künstlichen Gewebe festzulegen, Steuerdaten zu erzeugen und diese an die erste Lasereinrichtung oder die weitere Lasereinrichtung zu übermitteln, mit denen ein Blank erzeugbar ist, das durch eine im Zentrum des Blanks befindliche Korrekturzone, eine um diese herum angeordnete Übergangszone und eine um diese herum angeordnete Randzone, die zur späteren Abtrennung vor einer Einführung der Lamelle in die Hornhaut des Auges vorgesehen ist, definiert ist, und dieses Blank der Spender-Hornhaut bzw. dem künstlichen Gewebe entnommen und auf einem Halter fixiert werden kann, zur Bearbeitung mit der zweiten Lasereinrichtung.

In einer zweiten vorteilhaften Variante ist die Planungseinrichtung eingerichtet, Schnittflächen derart in der Spender-Hornhaut oder dem künstlichen Gewebe festzulegen, Steuerdaten zu erzeugen und diese an die erste Lasereinrichtung oder die weitere Lasereinrichtung zu übermitteln, mit denen ein Blank erzeugbar ist, das durch eine im Zentrum des Blanks befindliche Korrekturzone und eine um diese herum angeordnete Übergangszone definiert ist, und dieses Blank in der ursprünglichen Spender-Hornhaut oder dem künstlichen Gewebe mit der zweiten Lasereinrichtung weiterbearbeitet werden kann.

Auch in dieser zweiten Variante ist es von weiterem Vorteil, wenn die Planungseinrichtung eingerichtet ist, Schnittflächen derart in der Spender-Hornhaut oder dem künstlichen Gewebe festzulegen, Steuerdaten zu erzeugen und diese an die erste Lasereinrichtung oder die weitere Lasereinrichtung zu übermitteln, mit denen ein Blank erzeugbar ist, das zudem eine um die Übergangszone herum angeordnete Randzone, die zur späteren Abtrennung vor einer Einführung der Lamelle in die Hornhaut des Auges vorgesehen ist, aufweist.

Typische Größen der entsprechenden Zonen sind:
- für den Durchmesser der Korrekturzone: 3 mm bis 8 mm
- für die Breite der Übergangszone (radial): minimal 10µm, bevorzugt zwischen 50 µm und 2 mm
- für die Breite der Randzone: 0 bis 1 mm
- für die Randdicke: 1 µm bis 50 µm. Die Randdicke ist dabei die zu erreichende Dicke insbesondere im äußeren ringförmigen 10µm-Randbereich der Lamelle.

Dabei sind Korrekturzone, Übergangszone und Randzone in der Regel kreisförmig bzw. kreisringförmig. Es ist jedoch - je nach patientenspezifischen Gegebenheiten auch eine elliptische oder eine unregelmäßige Form möglich. Diese kann auch zum Zweck der winkelrichtigen Positionierung der Lamelle in der Hornhaut des Auges verwendet werden.

Dabei ist anzumerken, dass es derzeit keine Technologie zur Erzeugung einer definierten Randgeometrie bei einer patientenspezifisch geformten Lamelle gibt. Die im experimentellen Verfahren verwendeten Femtosekunden-Laserschnitte zur Erzeugung des zirka zylindrischen Blanks (das genaugenommen ein Kappe einer Kugelschale beschreibt) und die anschließende Excimer-Laserbearbeitung zu einer patientenspezifisch geformten Lamelle können noch keine präzise Randgeometrie erzeugen. Man kann davon ausgehen, dass - ohne die hier beschriebenen besonderen Maßnahmen - beispielsweise allein die Randdicke zufällig um bis zu 30 µm oder bis zu 100% vom Zielwert abweicht. Eine erfindungsgemäße Randzone zur späteren Abtrennung ist beispielsweise auch deswegen von Vorteil.

Die für die patientenspezifische Anpassung mittels Excimer-Lasereinrichtung als zweite Lasereinrichtung verwendeten Zernike-Polynome bis zur 6. Ordnung stellen eine erhebliche Einschränkung für das Verfahren dar. Zwar besteht bei einer in einer Excimer-Lasereinrichtung, wie beispielsweise dem MEL, bisher bereits existierenden topografiegeführten Behandlung eine Zernike-Zerlegung 10. Ordnung, jedoch ist diese Behandlungsmethode bisher noch nicht ex-vivo angewendet worden und mit dem bisher praktizierten Vorgehen auch nicht zugänglich. Es gibt also derzeit keine andere erprobte Technologie zur Ansteuerung einer Excimer-Lasereinrichtung bei der ex-vivo-Bearbeitung einer Lamelle.

Zwar wurde bereits Ende der 80-er Jahre das Ziel formuliert, Hornhaut ex-vivo mit einem Excimer-Laser zu bearbeiten und es gab auch einen praktischen Versuch der Umsetzung (P. Homolka et al., Das Excimer Laser Corneal Shaping-(ELCS)System. Elektrotechnik und Informationstechnik, 117. Jg. (2000)), doch setzte sich dieser Ansatz nicht in der klinischen Praxis durch.. Die Ursache für dieses Scheitern lag einerseits in der noch unreifen Excimer-Lasertechnologie, andererseits aber auch daran, dass moderne diagnostischen Möglichkeiten wie hochauflösende OCT-Geräte noch gar nicht vorhanden waren. Zudem standen zur Erzeugung von Rohlingen nur mechanische Mikrokeratome zur Verfügung, deren Bearbeitungsgenauigkeit eine Größenordnung hinter heutigen Femtosekunden-Lasersystemen zurückblieb. Somit kam zu der durch die Excimer-Technologie verursachten Rauigkeit der Transplantate von ca. 10 µm noch die Unsicherheit über die genaue Ausgangsform der bearbeiten Spenderhornhaut. Der daraus resultierende Mangel an Genauigkeit verhinderte die praktische Anwendung der erzeugten Transplantate.

Derzeit verwendet man bei sIALK eine Lamelle mit einer Randdicke von mindestens 30 µm. Das ist im Hinblick auf die mechanische Festigkeit und Manipulierbarkeit der Lamelle sinnvoll, führt aber trotz Vakanz zur Entstehung eines ringförmigen Hohlraums an der Berührungsstelle von Stroma, Lamelle und Cap. Ein Hohlraum füllt sich zwar mit Gewebsflüssigkeit, doch ist er unphysiologisch und soll möglichst vermieden werden. Im Stand der Technik gibt es also keine Technologie zur Erzeugung einer speziellen Randgeometrie der Lamelle.

Eine präzise Geometrie kann mit einer Excimer-Lasereinrichtung nur schwer hergestellt werden, wenn die Ortsfrequenzen hoch sind im Vergleich zum Strahldurchmesser. Die Herstellung eines einfachen zylindrischen Blanks mit einer Femtosekunden-Lasereinrichtung Laser zeigt bereits, dass mit einer Femtosekunden-Lasereinrichtung grundsätzlich die Möglichkeit besteht, präzise Kanten zu erzeugen. Deswegen wird eine Kombination von Bearbeitungsschritten mit einer Femtosekunden-Lasereinrichtung als vorbearbeitende zweite Lasereinrichtung und einer Excimer-Lasereinrichtung als nachbearbeitende zweite Lasereinrichtung vorgeschlagen, wobei jedoch die hier beschriebenen und folglich entsprechend zu planenden und in Steuerdaten umzusetzenden Bearbeitungsprinzipen zu beachten sind.

Die Planungseinrichtung zum Erzeugen von Steuerdaten für ein Behandlungssystem zur refraktiven Chirurgie, insbesondere zur Keratoplastie ist beispielsweise insbesondere eingerichtet, die Steuerdaten für die zweite Lasereinrichtung derart zu erzeugen, dass eine definierte Randgeometrie bei der patientenspezifisch geformten Lamelle erreicht wird, wobei
- im Fall eines Pocket-Schnittes, und damit ohne Erzeugung einer Vakanz, die Randdicke maximal 30µm, bevorzugt zwischen 5µm und 15µm, beträgt, oder
- im Fall einer Entnahme eines Hornhaut-Volumens und damit der Erzeugung einer Vakanz die Randdicke der Lamelle an die Vakanz-Geometrie angepasst ist.

Sie ist vorteilhaft weiterhin eingerichtet, eine im Wesentlichen ringförmige Übergangszone am Rande der Lamelle zu bestimmen, innerhalb der die Randdicke allmählich in ein patientenspezifisches Dickenprofil übergeht, und die Steuerdaten so erzeugt werden, dass keine Bearbeitung des Randes der Lamelle durch die zweite Lasereinrichtung erfolgt.

Der Rand der Lamelle sollte deshalb im Wesentlichen schon während der Erzeugung des Blanks beispielsweise mittels einer Femtosekunden-Lasereinrichtung, als der vorbearbeitenden ersten oder weiteren Lasereinrichtung, nach den Vorgaben erzeugt werden, während die eigentliche Bearbeitung / Individualisierung der Lamelle vor der Implantation in ein Empfängerauge beispielsweise mittels einer Excimer-Lasereinrichtung als nachbearbeitender zweiter Lasereinrichtung erfolgt.

Das Implantat weist bei Betrachtung in axialer Richtung eine vorzugsweise runde zentrale Korrekturzone auf. In dieser Zone wird es so geformt, dass es die angestrebte Korrektur beispielsweise eines Dickenprofils der Hornhaut oder einer Topografie oder einer Wellenfront möglichst gut erreicht. Die Korrekturzone wird von einer ringförmigen Übergangszone umgeben, welche dazu dient, einen stetigen Übergang der Form am Rand der Korrekturzone hin zur Randzone zu erzielen. Diese ebenfalls ringförmige Zone erstreckt sich mit einer möglichst konstanten Höhe (Dicke) bis zum äußeren Rand des Implantats. Es ist natürlich auch möglich, diese Randzone weg zu lassen, wodurch allerdings der damit verbundene Vorteil verschwindet. Denn der Zweck der Randzone ist es, die Auswirkung eines Zentrierfehlers bei der sequenziellen Laser-Bearbeitung auf die äußere Randdicke des Implantats möglichst zu vermeiden oder wenigstens zu verringern. Ein weiterer Zweck einer (extensiven) Randzone besteht im Schutz der der näheren Randzone einer (fast fertigen) Lamelle bis zu deren Implantation, wie unten noch ausgeführt wird.

Dabei ist festzustellen, dass die Maßnahmen zu einem definierten Rand der Lamelle und einem Temperaturregime zwar besonders effektiv sind, wenn die Gerätekoordinatensysteme der erste Lasereinrichtung, der die Charakterisierungseinrichtung und der zweiten Lasereinrichtung über die Planungseinrichtung mittels Registrierung gekoppelt und die zugeführten zweiten Messdaten der Lamelle eindeutig zu den Gerätekoordinatensystemen registrierbar sind. Aber auch ohne eine solche Kopplung und Registrierung zueinander tragen die Maßnahmen zu einem definierten Rand der Lamelle und einem Temperaturregime während der Bearbeitung zu einer gegenüber dem Stand der Technik verbesserter Präzision und insbesondere einer Wiederherstellung einer normalen Hornhautgeometrie mit gegenüber dem Stand der Technik verbesserter optischer Funktion der Hornhaut bei.

Wie schon beschrieben, wird eine ringförmige Übergangszone am Rand vorgesehen, deren radiale Breite mindestens 10 µm beträgt, bevorzugt zwischen 50 µm und 2mm, besonders bevorzugt zwischen 50µm und 500µm. Innerhalb dieser Übergangszone wird die Randdicke allmählich in das patientenspezifische Dickenprofil überführt. Im einfachsten Fall geschieht dies durch eine lineare Verbindung von der Randdicke D(r=r_max, Phi) zu D(r=r_max-Randbreite, Phi). Weitere Ausgestaltungen, sind für den Fachmann naheliegend, auch eine Glättung in Phi ist möglich.

Die Randdicke und das Profil innerhalb der Übergangszone wird beispielsweise durch das Zusammenwirken von einer Femtosekunden-Lasereinrichtung als vorbearbeitende erste oder weitere Lasereinrichtung und einer Excimer-Lasereinrichtung als nachbearbeitende zweite Lasereinrichtung erreicht. Insbesondere wird in der Erfindung die Excimer-Bearbeitung so durchgeführt, dass keine Excimer-Ablation auf dem Rand des Blanks erfolgt. Denn es soll einerseits verhindert werden, dass die mit der Femtosekunden-Lasereinrichtung präzise erzeugte Randdicke durch die Excimer-Lasereinrichtung verändert wird und andererseits darf, wenn das Blank zur Bearbeitung durch die Excimer-Lasereinrichtung auf einem Halter fixiert ist, keine Ablation des Blank-Halters erfolgen, weil dadurch die Lamelle kontaminiert werde könnte. Um diesen Aspekt weiter zu verbessern wird in einer Variante die Übergangszone ganz oder teilweise durch die Femtosekunden-Lasereinrichtung als vorbearbeitende erste oder weitere Lasereinrichtung erzeugt, beispielsweise indem eine kegelförmige Grundgeometrie der Randzone des Blanks erzeugt wird.

Die Ansteuerung einer Excimer-Lasereinrichtung bei der ex-vivo-Bearbeitung einer Lamelle erfolgt derzeit mit einem Schussmuster, welches die Zernike-Entwicklung des Differenzprofils in einzelne Ablationsvolumina zerlegt. Derzeit wird diese Entwicklung nur bis zur 6. Ordnung des Polynoms durchgeführt. Die Entwicklung über die 6. Ordnung hinaus ist bei diesen ex-vivo-Bearbeitungen jedoch sinnvoll, um feinere Strukturen erzeugen zu können. Es ist allerdings nicht zwingend erforderlich, eine solche Entwicklung vorzunehmen. Stattdessen kann das Differenzprofil direkt in einzelne Ablationsvolumina zerlegt werden (Schusszerlegung). In jedem Fall ist es entscheidend, die Bearbeitung so durchzuführen, dass die Temperatur des Blanks 40° C nicht übersteigt. Dafür werden die Schussverteilung und Laserfrequenz des Scanning-Spot-Lasers entsprechend angepasst.

Weiterhin ist zum Stand der Technik, seinen Problemen und den daraus resultierendenerfindungsgemäßen Maßnahmen folgendes anzumerken:
Die mit der Regularisierung der Hornhautdicke verbundene Verbesserung des Brillenvisus (CDVA) ist bereits ein gutes Ergebnis für den Patienten. Grundsätzlich besteht zur Erreichung eines guten unkorrigierten Visus (UCVA) auch die Möglichkeit zur Durchführung einer refraktiven Korrekturbehandlung, doch wäre eine Photorefraktive Keratektomie (PRK) immer mit dem Verlust der Bowman-Membran verbunden, was in einem biomechanisch instabilen Auge eine medizinisch fragwürdige Option ist. Eine Laser-in-situ-Keratomileusis (LASIK) verbietet sich aufgrund der noch größeren biomechanischen Implikation und eine Femtosekunden-Lentikelextraktion (Small Incision Lenticle Extraction, SMILE)-Behandlung wäre chirurgisch kaum durchführbar. Eine Vorderkammerlinse wäre denkbar, aber sie stellt bei einem solchen Patienten ein hohes Risiko dar, gleiches gilt für eine Intraokularlinse (IOL), die in diesem Fall eine nichtgetrübte Linse ersetzen würde (clear lens exchange).

Solange man nur die Regularisierung der Hornhautdicke (Pachymetrie) anstrebt, erzielt man einen Zustand, in dem das Patientenauge mit Hilfe einer Sehhilfe (Brille, Kontaktlinse) einen guten Visus hat. Strebt man einen noch besseren Zustand an, dass also beispielsweise bereits ohne Brillenkorrektur ein guter Visus erreicht werden kann, so erfordert dies zusätzliche Maßnahmen. Grundzüge zur Lösung dieses Problems sind in Teilen bereits in DE 10 2007 019 815 A1 bzw. WO 2008/131888 A1 formuliert, konkrete oder gar vorteilhafte Ausgestaltungen sind bisher aber nicht bekannt.

Deshalb ist eine vorteilhafte Planungseinrichtung eingerichtet, die Steuerdaten für die zweite Lasereinrichtung so zu erzeugen, dass dem Blank eine Brechkraft und/oder ein Astigmatismus aufgeprägt wird.

Für ein präziseres Zusammenspiel zwischen zweiter Lasereinrichtung mit der ersten bzw. weiteren Lasereinrichtung, die zur Vorbereitung des Blanks genutzt wurde, ist einer besonders vorteilhafte erfindungsgemäße Planungseinrichtung eingerichtet, in der Übergangszone und/oder der Randzone die Lage von Kalibriermarken zu definieren und Steuerdaten für die ersten Lasereinrichtung oder die weiteren Lasereinrichtung zu erzeugen mit denen diese Kalibriermarken während einer Bearbeitung mit der ersten Lasereinrichtung oder der weiteren Lasereinrichtung einbringbar sind, wobei die Kalibriermarken derart definiert sind, dass sie als ein- oder mehrfache Orientierung während einer Bearbeitung des Blanks mittels der zweiten Lasereinrichtung nutzbar sind.

Insbesondere ist es hilfreich, wenn die erfindungsgemäße Planungseinrichtung die Kalibriermarken so definiert, dass sie mehrfach übereinander und/oder zueinander versetzt und/oder in verschiedenen Höhen im durch die zweite Lasereinrichtung zu bearbeitendem Blank angeordnet sind.

Als erfindungsgemäßer optionaler Schritt beim Schneiden des Blanks in der Spenderhornhaut oder dem künstlichen Gewebe beispielsweise mittels einer Femtosekunden-Lasereinrichtung ist also jeweils die Einbringung von Kalibrierschnitte möglich, insbesondere vorteilhaft sind dabei Kalibrierschnitte in einem Excavation-Volumen, das hier im Folgenden noch beschrieben wird.

Als weiterer optionaler Schnitt beim Bearbeiten mit beispielsweise einer ablatierenden Excimer-Lasereinrichtung als zweite Lasereinrichtung ist dann jeweils eine Unterbrechung der Bearbeitung möglich, gefolgt von einer Möglichkeit zur Fortsetzung nach visueller Kontrolle durch den Anwender. Hierbei prüft der Anwender insbesondere, ob eine oder mehrere der Kalibriermarken durch die Excimer-Behandlung bereits getroffen wurden und entscheidet entsprechend über eine Fortsetzung des Abtrags. Dieses Verfahren kann auch mittels entsprechender Bildaufnahme- und Bildverarbeitungsvorrichtung automatisiert erfolgen, wobei diese Automatisierung durch einen Controller mit entsprechender Software erfolgt.

Generell kann die Bearbeitung mit dem ablatierenden Excimer-Laser in verschiedenen Phasen erfolgen, beispielsweise einer ersten Phase zur Entfernung des Epithels und einer zweiten Phase für die Bearbeitung des stromalen Gewebes.

Im Konkreten werden die Kalibriermarken beispielsweise, wie in einigen Ausführungsbeispielen gezeigt, in das Excavation-Volumen eingebracht. Wenn dies in einem Arbeitsgang mit der Erzeugung der anderen Schnittfläche beispielsweise mit einer Femtosekunden-Lasereinrichtung als erster bzw. weiterer Lasereinrichtung erfolgt, können diese Kalibriermarken bezüglich der anderen Schnittflächen sehr genau positioniert werden. Eine Genauigkeit von besser als 1 µm axial wie lateral ist erreichbar. Erreicht dann bei der Bearbeitung des Blanks mit beispielsweise einer Excimer-Lasereinrichtung als zweite Lasereinrichtung der Excimer-Abtrag eine solche Kalibriermarke, welche im Gewebe für einige Zeit als mit Gasblasen gefüllte Schnittfläche erhalten bleibt, so zeigt sich eine sehr charakteristische Änderung der optischen Erscheinung der jeweiligen Kalibriermarke. Auf diese Weise kann durch eine fachmännische geometrische Gestaltung von Kalibriermarken und Ablationsprofil, insbesondere im Excavation-Volumen eine optimierte Überlagerung der beiden Laserbearbeitungen erzielt werden, womit sich beispielsweise aufwandgering eine präzise Randdicke herstellen lässt.

Wie schon angedeutet, ist es weiterhin vorteilhaft, wenn eine Planungseinrichtung eingerichtet ist, ein Bearbeitungsprofil für die zweite Lasereinrichtung derart zu definieren und die Steuerdaten zu bestimmen, dass das Profil der im Zentrum des Blanks befindlichen Korrekturzone und der um diese herum angeordnete Übergangszone mittels der zweiten Lasereinrichtung erzeugbar ist und in der um dies Übergangszone herum angeordneten Randzone eine Excavation erzeugbar ist
- zur Entnahme einer aus der Spender-Hornhaut oder dem künstlichen Gewebe herausgearbeiteten Lamelle, oder
- zur Weiterbearbeitung des Blanks auf einem Halter derart, dass der Halter durch einen Bearbeitungslaserstrahl der zweiten Lasereinrichtung nicht getroffen werden kann.

Die Erzeugung einer Excavation innerhalb des Verfahrens zur Herstellung des Implantats in Form einer patientenspezifischen Lamelle ist ein wichtiger Teil der Erfindung. Als Excavation wird die Schaffung eines normalerweise ringförmigen Hohlraums um die äußere Peripherie des Implantats bezeichnet. Dieser grabenförmige Hohlraum dient dazu, dem Chirurgen die Freilegung des Implantats im Zuge seiner Abtrennung zu erleichtern, oder bei einem bereits freigelegten Implantat nicht benötigtes Material schonend von der Peripherie des Implantats zu entfernen. Auf diese Weise wird verhindert, dass eine Beschädigung der extrem feinen Randstruktur des Materials vor der Einführung der Lamelle in eine entsprechende Schnittfläche bzw. Struktur in der Hornhaut eines Empfängerauges erfolgt. **In** den Abbildungen der Ausführungsbeispiele werden einige charakteristischen Merkmale einer Excavation weiter verdeutlicht.

Dabei sind typische Werte für die Breite einer Excavation (radial): 500 µm bis 3 mm.

Dabei sollte der Durchmesser eines ggf. durchgeführten zusätzlichen Excavation-Schnitts mindestens 100 µm größer als der Durchmesser des eigentlichen Implantats, also der finalen Lamelle, sein.

In diesem Zusammenhang ist ebenfalls zu erwähnen, um eine reale Größenordnung zu geben, dass für die Erzeugung der oben beschriebenen Kalibriermarken Kalibrierschnitte in einer bis zu hundert Flächen erzeugt werden.

Des Weiteren ist es vorteilhaft, wenn die erfindungsgemäße Planungseinrichtung eingerichtet ist, für die Erzeugung der Steuerdaten einen definierten Ausgangshydratationszustand des Blanks bzw. der Lamelle ex-vivo und die Änderung des Hydratationszustands der Lamelle während bzw. nach der Implantation, bevorzugt mittels eines konstanten Expansionsfaktors, zu berücksichtigen.

Das Problem unterschiedlicher Hydratation ist bei der Hornhauttransplantation bereits bekannt und wird durch spezifische Behandlung der Transplantate vermindert. Es tritt im Zusammenhang mit sILAK jedoch erstmals in dieser Form auf, denn bisher werden Hornhäute nicht ex-vivo z.B. mit einer Excimer-Lasereinrichtung patientenspezifisch geformt. Zum Schwellen und Schrumpfen kommt hierbei noch die durch die Hydratation veränderte Abtrags-Effizienz der Excimer-Lasereinrichtung.

Bei der Analyse der bisherigen Vorgehensweise ist aufgefallen, dass die Variabilität des Materials, insbesondere seines Hydratationszustandes, die Quelle erheblicher Fehler sein kann. Das war bisher nicht klar und erfordert geeignete Maßnahmen, um letztendlich die Geometrie der Lamelle in der Empfängerhornhaut, insbesondere ihre Dicke, präziser vorherbestimmen zu können.

Eine zusätzliche Möglichkeit zur verbesserten Kontrolle des Hydratationszustands ist die Nutzung eines künstlichen Gewebematerials mit wohldefinierten Parametern, aus dem dann die zu implantierende Lamelle kontrollierter mittels der zweiten Lasereinrichtung herausgearbeitet werden kann - als Ersatz eines natürlichen Transplantationsmaterials.

Das Blank bzw. die Lamelle können zudem zur besseren Handhabbarkeit mit einem Farbstoff eingefärbt werden, der nach der Implantation der Lamelle nach und nach wieder verschwindet.

Die Aufgabe wird weiterhin gelöst durch ein erfindungsgemäßes Behandlungssystem für die refraktiven Chirurgie, insbesondere für die Keratoplastie, das
- eine erste Lasereinrichtung zur Erzeugung mindestens einer Schnittfläche in einer Hornhaut eines Auges, vorzugsweise eine Femtosekunden-Lasereinrichtung,
- eine zweite Lasereinrichtung zur Bearbeitung eines Blanks zur patientenspezifisch geformten Lamelle, vorzugsweise eine Excimer-Lasereinrichtung,
- mindestens eine Charakterisierungseinrichtung, vorzugsweise eine OCT-Einrichtung, und
- eine hier beschriebene erfindungsgemäße Planungseinrichtung umfasst. Vorteilhaft ist ein erfindungsgemäßes Behandlungssystem, das eine weitere Lasereinrichtung zur Erzeugung bzw. Vorbearbeitung des Blanks, vorzugsweise eine Femtosekunden-Lasereinrichtung, aufweist.

In der Ausgestaltung des erfindungsgemäßen Behandlungssystems enthält die zweite Lasereinrichtung einen Halter zur Fixierung des Blanks während der Bearbeitung.

Eine besondere Ausgestaltung des erfindungsgemäßen Behandlungssystems weist eine Temperaturregelungseinrichtung auf, vorzugsweise derart, dass eine Temperatur des Blanks so abgesenkt werden kann, dass das Blank im gefrorenen Zustand bearbeitbar ist.

Für die Bearbeitung des Blanks kann dieses dann gekühlt werden. Die verringerte Temperatur führt während der Bearbeitung durch Reduzierung der Austrocknung und/oder Verringerung von temperaturbedingten Materialveränderungen zu einer präziseren Bearbeitung. Letzteres spielt insbesondere dann eine Rolle, wenn die Bearbeitung mit hoher Laserfrequenz erfolgt und entsprechend schnell und viel Energie in den Blank eingetragen wird. Dieser Energieeitrag ist nicht immer auf die unmittelbare Wechselwirkungszone der Laserstrahlung mit dem Material beschränkt, sondern kann durch Wärmeübertragung und Wärmeleitung auch die Umgebung der Wechselwirkungszone umfassen. Da insbesondere biotechnologisch hergestellte (künstliche) Materialien mitunter empfindlich auf Temperaturerhöhung reagieren, sich also ungünstig verändern, kann in einer Ausprägung des erfindungsgemäßen Behandlungssystems wie auch eines erfindungsgemäßen Verfahrens die Kühlung des Blanks Abhilfe schaffen.

In einer bevorzugten Ausgestaltung wird dabei die Temperatur des Blanks, insbesondere eines Blanks, das zur Bearbeitung auf einem Halter fixiert ist, bis etwa zum Taupunkt der Umgebung abgesenkt. Das bedeutet beispielsweise, dass bei einer Raumluft-Temperatur von 20°C und 50% Luftfeuchte eine Oberflächentemperatur von ca. 9 °C eingestellt wird.

In einer besonders bevorzugten Ausgestaltung wird die Temperatur des Blanks auf dem Halter so weit abgesenkt, dass es gefriert. Eine derartige Verringerung der Temperatur des Blanks unter seinen Gefrierpunkt kann durch einen entsprechend kalten Halter auch in normaler Raumluftumgebung erzielt werden. Die Laserbearbeitung erfolgt dann am gefrorenen Blank. Die Temperatur des Blanks kann beispielsweise auf unter 0°C, unter -5°C oder unter -10°C abgesenkt werden. Der Vorteil einer sehr tiefen Temperatur besteht darin, dass die Wechselwirkung der Laserstrahlung mit nicht ablatiertem Material verringert wird. Die Betauung bzw. Bereifung des Materials wird durch einen schnellen Prozess (z.B. komplette Laserbearbeitung in weniger als 1 Minute) minimiert oder durch einen entsprechend gestalteten Ablationsprozess kompensiert.

In einem speziellen erfindungsgemäßen Behandlungssystem weist dessen Temperaturregelungseinrichtung mindestens eine der folgenden Ausgestaltungen auf:
- eine aktive elektrische Kühlung mittels Peltier-Element,
- eine aktive Kühlung mittels eines eingebrachten Kühlmittels,
- eine aktive Kühlung durch einen Luftstrom,
- eine passive Kühlung durch Vorkühlung des Halters mit oder ohne dem darauf fixierten Blank,
- eine von der Umgebung abgetrennten Kammer zur Prozessierung des Blanks.

Der Halter kann also zum Zweck der Kühlung mit einem Kühlmechanismus ausgestattet sein. Bei diesem handelt es sich beispielsweise um eine aktive elektrische Kühlung mittels Peltier-Element. Denkbar ist aber auch eine aktive Kühlung mittels eines eingebrachten Kühlmittels (z.B. Stickstoff, Gylcerol, Ethanol) oder passive Vorkühlung in einer entsprechend kalten Umgebung (Kühlschrank). Die Kühlung kann geregelt erfolgen, wobei in einer entsprechenden Ausführung des Halters dessen Temperatur oder die Temperatur des Blanks überwacht wird.

Statt normaler Raumluft (23° C, 50% relative Luftfeuchte) kann das Blank auch mit einem Luftstrom beaufschlagt werden, der neben der üblichen Wirkung, dem Abtransport des Debris, eine Beeinflussung der Temperatur des Blanks bewirkt. Dies erfolgt durch eine Anpassung der Temperatur und/oder der Luftfeuchte. Alternativ kann statt Raumluft auch ein Schutzgas verwendet werden. Zur weiteren Verbesserung dieser Methode kann das Blank in einer von der Umgebung abgetrennten Kammer bearbeitet werden.

Als Richtwerte für eine konkrete Bearbeitung des Blanks auf einem Halter in der zweiten Lasereinrichtung, insbesondere einer Excimer-Lasereinrichtung kann mit folgenden Werten gearbeitet werden:
- für frostfreien Betrieb sollte die Temperatur des Halters ca. 1°C bis 20°C betragen.
- für "Frostbetrieb" sollte die Temperatur des Halters ca. -30°C bis -1°C betragen. Von weiterem Vorteil ist ein erfindungsgemäßes Behandlungssystem, das eine Einrichtung zur Überwachung der Temperatur des Blanks und/oder des Halters während der Bearbeitung des Blanks aufweist.

Weiterhin wird als Hintergrundinformation ein Planungsverfahren beschrieben, das die Erzeugung von Steuerdaten für ein Behandlungssystem für die refraktiven Chirurgie, insbesondere für die Keratoplastie, gemäß einer Kodierung der beschriebenen Planungseinrichtung realisiert.

Auch als Hintergrundinformation und nicht als Teil der beanspruchten Erfindung wird ein Verfahren der refraktiven Chirurgie, insbesondere der Keratoplastie, beschrieben, in dem mindestens eine Schnittfläche in der Hornhaut eines Auges erzeugt wird, eine Lamelle zur Einführung in die Hornhaut eines (Empfänger-)Auges mittels des beschriebenen Planungsverfahrens geplant, mit Hilfe der erzeugten Steuerdaten hergestellt und in die mindestens eine Schnittfläche, ggf. auch in eine durch die Schnittflächenausbildung entstandene Vakanz, in der Hornhaut des Auges eingeführt wird.

Dabei kann bei der Herstellung der Lamelle auf eine der folgenden Verfahrensvarianten zurückgegriffen werden.
Variante 1:
   1. Schneiden des Blanks in einer Spender-Hornhaut oder einem künstlichen Gewebe mittels einer ersten oder weiteren Lasereinrichtung, insbesondere einer Femtosekunden-Lasereinrichtung (auch fs-Laserkeratom genannt).
   2. Bearbeiten des Blanks in der Spender-Hornhaut oder dem künstlichen Gewebe mit einer zweiten Lasereinrichtung, insbesondere einer Excimer-Lasereinrichtung, dabei Erzeugung einer Korrekturzone, einer Übergangszone und einer Randzone, sowie ggf. Einbringung einer Excavation zum Freilegen des Implantats (also der nahezu fertigen Lamelle).
   3. Resektion des Implantats aus der Spender-Hornhaut oder dem künstlichen Gewebe.
Variante 2:
   1. Schneiden des Blanks in einer Spender-Hornhaut oder einem künstlichen Gewebe mittels einer ersten oder weiteren Lasereinrichtung, insbesondere einer Femtosekunden-Lasereinrichtung
   2. Resektion des Blanks aus der Spender-Hornhaut oder dem künstlichen Gewebe und Positionierung auf einem Halter.
   3. Bearbeiten des Blanks auf dem Halter mit einer zweiten Lasereinrichtung, insbesondere einer Excimer-Lasereinrichtung, dabei Erzeugung der Korrekturzone, der Übergangszone und der Randzone, sowie ggf. Einbringung einer Excavation.
   4. Optional: Trennung des Implantats (also letztlich der patientenspezifischen Lamelle von den Resten des Blanks
Variante 3:
   1. Positionierung des Blanks (aus einem Spender-Hornhaut-Material oder einem künstlichen Gewebematerial) auf einem Halter.
   2. Optional: Starten der Kühlung des Halters
   3. Bearbeiten des Blanks auf dem Halter mit einer zweiten Lasereinrichtung, insbesondere einer Excimer-Lasereinrichtung und optional mit einer ersten oder weiteren Lasereinrichtung, insbesondere einer Femtosekunden-Lasereinrichtung (fs-Laserkeratom), dabei Erzeugung der Korrekturzone, der Übergangszone und der Randzone. Einbringung einer Excavation.
   4. Optional: Trennung des Implantats vom Halter nach Erwärmung des Halters
Variante 4:
   1. Durchführung einer der Varianten 1 bis 3
   2. Trennung des Implantats vom Halter
   3. Flippen (also Umstülpen) des Implantats (Ein kugelschalenförmiges Volumen kann man "flippen"/umstülpen)
   4. Positionierung des Implantats auf dem Halter, wobei die bisherige Oberseite nun unten liegt
   5. Erneute Durchführung einer der Varianten 1 bis 3

Die vorliegende Erfindung soll nun anhand von Ausführungsbeispielen erläutert werden. Es zeigt:
- die Fig. 1a das Schema eines bevorzugten ersten erfindungsgemäßes Behandlungssystems mit einer ersten erfindungsgemäßen Planungseinrichtung, das nicht die exakten physischen Gegebenheiten wiederspiegelt.
- die Fig. 1b das Schema eines bevorzugten zweiten erfindungsgemäßes Behandlungssystems mit einer zweiten erfindungsgemäßen Planungseinrichtung,
- die Fig. 1c das Schema eines bevorzugten dritten erfindungsgemäßes Behandlungssystems mit einer dritten erfindungsgemäßen Planungseinrichtung.
- die Fig. 2a bis 2f verschiedene Bearbeitungsstufen wie auch Bearbeitungsvarianten eines Blanks in einer Spender-Hornhaut oder einem künstlichen Gewebe für einen Ablauf der Erzeugung eines Implantats/einer patientenspezifischen Lamelle wie beispielsweise mit Hilfe der erfindungsgemäßen Planungseinrichtung erzielbar - für die prinzipielle Variante der Bearbeitung des Blanks bis zur vollständigen Erzeugung der patientenspezifischen Lamelle innerhalb der Spender-Hornhaut bzw. dem künstlichen Gewebe.
- die Fig. 3a bis 3f verschiedene Bearbeitungsstufen wie auch Bearbeitungsvarianten eines Blanks in einer Spender-Hornhaut oder einem künstlichen Gewebe für einen Ablauf der Erzeugung eines Implantats/einer patientenspezifischen Lamelle wie beispielsweise mit Hilfe der erfindungsgemäßen Planungseinrichtung erzielbar - für die prinzipielle Variante der Weiterbearbeitung des Blanks mittels der zweiten Lasereinrichtung, hier eine Excimer-Lasereinrichtung, derart, dass das Blank der Spender-Hornhaut bzw. dem künstlichen Gewebe entnommen wird und auf einem Halter fixiert wird.
- die Fig. 4a bis 4c ein Implantat nach Bearbeitung mit den Lasereinrichtungen des erfindungsgemäßen Behandlungssystems und vor seiner Einführung in eine Schnittfläche der Hornhaut eines Empfänger-Auges.

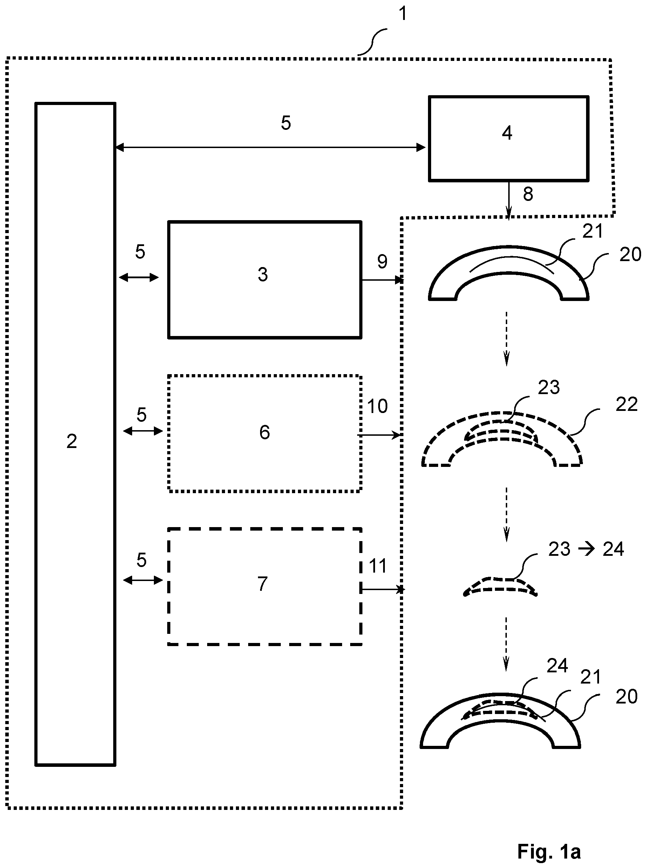

In allen Fig. 1a bis 1c umfasst das Behandlungssystem 1 eine Planungseinrichtung 2, eine Charakterisierungseinrichtung 4, die eingerichtet ist, mit einer Untersuchungsstrahlung 8 Messdaten über Parameter der Hornhaut 20 eines Auges zu erzeugen, eine erste Lasereinrichtung 3, die hier eine Femtosekunden-Lasereinrichtung ist und die eingerichtet ist, mittels eines fokussierten Femtosekunden-Laserstrahls 9 eine Vakanz oder, wie hier dargestellt, einen Pocket-Schnitt 21 in der Hornhaut 20 eines Empfängerauges zu erzeugen (die Einfallsrichtung des Strahls ist hier nicht dargestellt - der Fachmann kennt jedoch den optischen Aufbau entsprechender Einrichtungen).

Alle Charakterisierungs- und Lasereinrichtungen des Behandlungssystems 1 enthalten Schnittstellen 5 zur Planungseinrichtung 2.

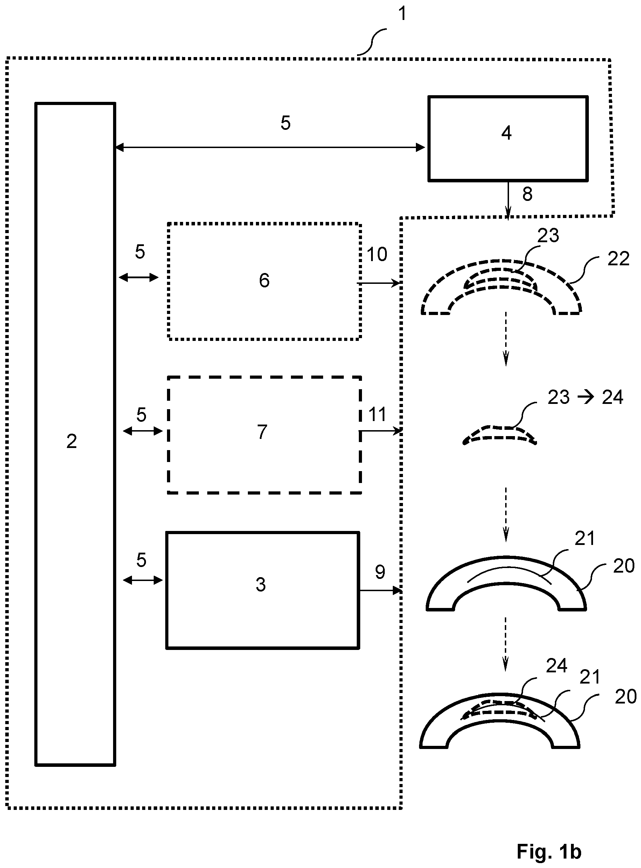

Die Fig 1a und 1b umfassen weiterhin eine vorbehandelnde weitere Lasereinrichtung 6, die ebenfalls eine Femtosekunden-Lasereinrichtung ist, wobei die erste Femtosekunden-Lasereinrichtung 3 und die vorbehandelnde weitere Femtosekunden-Lasereinrichtung ein und dieselbe Einrichtung oder aber zwei unterschiedliche Lasereinrichtungen sein können. Die vorbehandelnde weitere Lasereinrichtung schneidet mit einem fokussierten Femtosekunden-Lasterstrahl 10 ein Blank 23 aus der Hornhaut eines Spenderauges 22. Die Figuren 1a bis 1c umfassen weiterhin eine nachbehandelnde zweite Lasereinrichtung, hier eine Excimer-Lasereinrichtung 7, die mit einer Excimer-Laserstrahlung 11 die zu implantierende Lamelle 24 aus dem Blank 23 herausarbeitet, die letztlich dann im Pocket 21 der Hornhaut des Empfängerauges 20 implantiert wird.

Die Planungseinrichtung 2, ist eingerichtet, die Gerätekoordinatensysteme der beteiligten Lasereinrichtungen 3, 6, 7 und Charakterisierungseinrichtungen 4 mittels Registrierung zu koppeln und die zugeführten Messdaten der zu implantierenden Lamelle 23 eindeutig zu den Gerätekoordinatensystemen zu registrieren.

Während in der Fig. 1a zunächst der Pocket-Schnitt 21 in der Hornhaut des Empfängerauges 20 erzeugt wird, und erst hernach das Blank 23 im Spenderauge erzeugt und aus diesem entfernt wird, um es anschließend zur Lamelle 24 zu formen wie es bevorzugt gehandhabt werden soll, erfolgt in der Fig. 1b zunächst die vollständige Bearbeitung des Blanks 23 zur Lamelle 24. Erst hernach erfolgt dann ein Pocket-Schnitt 21 in der Hornhaut des Empfängerauges 20 zur Vorbereitung der Implantation.

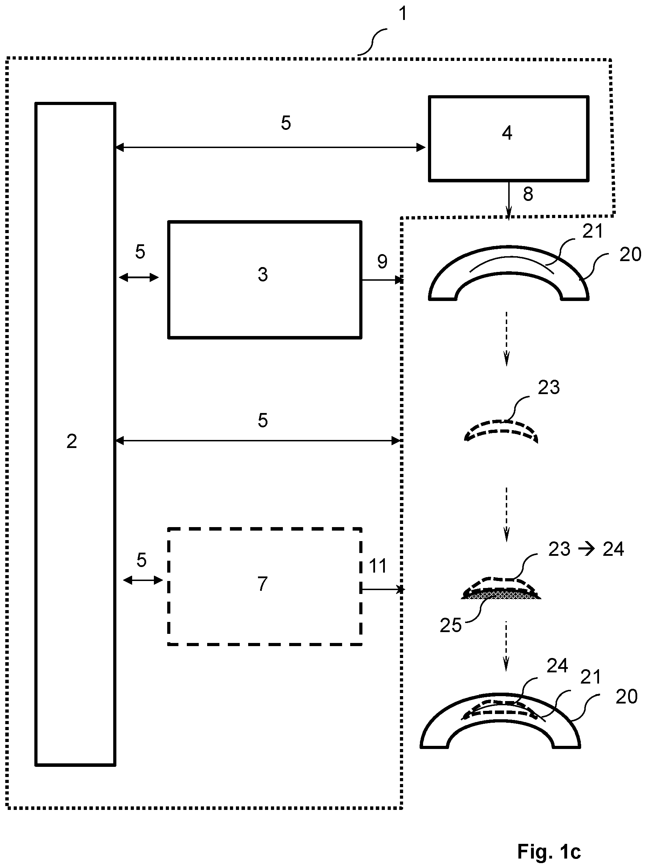

In der Fig. 1c wiederum wird mit standardisierten Blanks 23 gearbeitet (vorzugsweise aus einem künstlichen Gewebematerial), die dann nur noch zur Lamelle 24 nachbearbeitet werden. Gezeigt ist hier jedoch auch die Fixierung des Blanks 23 auf einem Halter 25 während seiner Bearbeitung mittels der Exminer-Lasereinrichtung 7 als zweite Lasereinrichtung. Der Halter wird während der Bearbeitung des Blanks gekühlt.

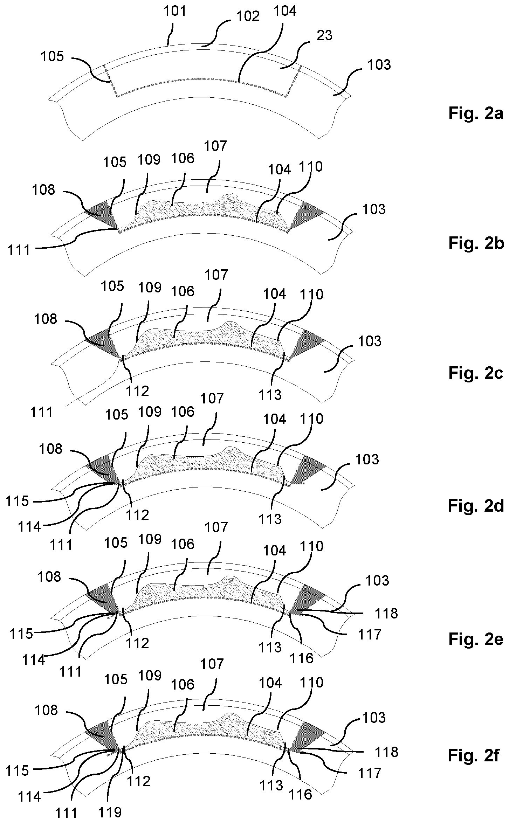

Die Fig. 2a bis 2f zeigen verschiedene Bearbeitungsstufen wie auch Bearbeitungsvarianten eines Blanks 23 in einer Spender-Hornhaut oder einem künstlichen Gewebe für einen Ablauf der Erzeugung eines Implantats/einer patientenspezifischen Lamelle 24 wie beispielsweise mit Hilfe der erfindungsgemäßen Planungseinrichtung 2 erzielbar - für die prinzipielle Variante der Bearbeitung des Blanks 23 bis zur Erzeugung der patientenspezifischen Lamelle 24 innerhalb der Spender-Hornhaut bzw. dem künstlichen Gewebe.

Dabei ist in der Fig. 2a eine Spender-Hornhaut mit ihren einzelnen Schichten, der Epithelschicht 101, der Bowman-Membran 102 und dem Cornealen Stroma 103 dargestellt. Zudem werden die Schnitte gezeigt, die das Blank 23 ganz grundsätzlich von der Spender-Hornhaut separieren: Der lamelläre Schnitt ("lamellar cut") 104 und der Randschnitt ("side cut") 105.

In der Fig. 2b ist des Weiteren das zu erzeugende Implantat 106 in Form der patientenspezifischen Lamelle 24 und daraus folgend das Ablationsvolumen 107, das mit der zweiten Lasereinrichtung 7 ablatiert werden soll, dargestellt. Weiterhin aufgezeigt ist die Excavation 108, die später der vereinfachten Separierung des Implantats 106 aus der Spender-Hornhaut (bzw. einem künstlichen Gewebe) dienen soll. Sichtbar in der Fig. 2b sind zudem die beiden äußeren Begrenzungen der Korrekturzone 109, 110, sowie die obere äußere Begrenzung des Implantats 111, die den Schnittpunkt von Randschnitt 105 und finaler Implantat-Oberseite darstellt.

In der Fig. 2c ist neben den in den Fig. 2a und 2b genannten Bereichen, Schnitten und Markierungen die äußere Begrenzung der Übergangszone 112, 113 dargestellt.

Die Fig. 2d zeigt neben den bereits in den Fig. 2a bis 2c genannten Bereichen, Schnitten und Markierungen Kalibriermarken 114. Mit deren Hilfe kann dann beispielsweise die Form der Excavation 108 gegenüber der in den Fig. 2b und 2c gezeigten Form 115 der Excavation verändert werden: Durch Einbringen von Kalibriermarken 114 mittels der ersten 3 bzw. weiteren Lasereinrichtung 6 (hier also der Femtosekunden-Lasereinrichtung) wird hiernach das Abtragungsprofil in den Randbereichen während des Ablationsprozesses mittels der zweiten Lasereinrichtung 7 (hier der Excimer-Lasereinrichtung) gesteuert.

Die Fig. 2e zeigt neben den bereits erwähnten Kalibriermarken 114 weitere Kalibriermarken: Kalibriermarken über dem Implantat in dessen Randbereich 116 und Kalibriermarken neben dem Implantat, sowie gestapelte Kalibriermarken 117, 118.

In der Fig. 2f ist zudem noch ein Randschnitt 119 mit der ersten 3 bzw. weiteren Lasereinrichtung 6, der Femtosekunden-Lasereinrichtung, geplant, der damit die Höhe des Implantats 106 in dessen Randzone, insbesondere der patientenspezifische Lamelle 24 als Endprodukt der Planung und Bearbeitung des Blanks 23, sicherer festlegt, als wenn diese Höhe allein durch die Ablation mittels der zweiten Lasereinrichtung 7, der Excimer-Lasereinrichtung, erzeugt wird.

In diesen Varianten der Fig. 2a bis 2f wird also die patientenspezifische Lamelle 24 in der Spender-Hornhaut aus einem Blank 23 herausgearbeitet. Erst nach dieser Bearbeitung wird das Implantat 106 dieser Spender-Hornhaut bzw. dem künstlichen Gewebe entnommen.

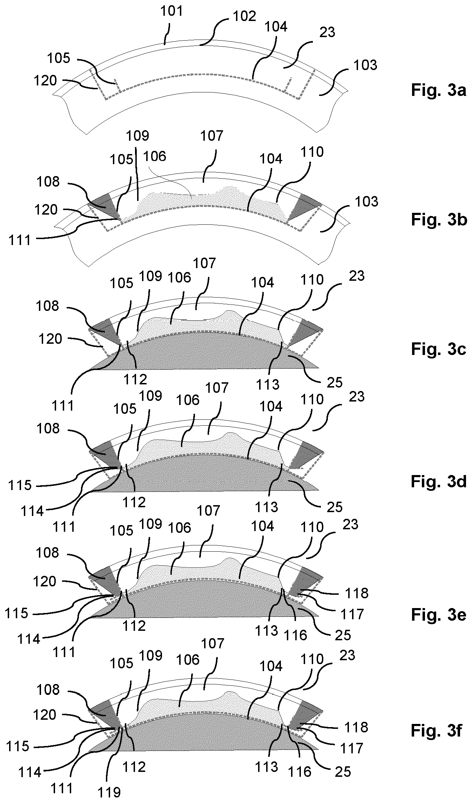

Die Fig. 3a bis 3f zeigen verschiedene Bearbeitungsstufen wie auch Bearbeitungsvarianten eines Blanks 23 in einem künstlichen Gewebe bzw. einer Spender-Hornhaut für einen Ablauf der Erzeugung eines Implantats/einer patientenspezifischen Lamelle 24 wie beispielsweise mit Hilfe der erfindungsgemäßen Planungseinrichtung 2 erzielbar - für die prinzipielle Variante der Weiterbearbeitung des Blanks mittels der zweiten Lasereinrichtung 7, hier eine Excimer-Lasereinrichtung, derart, dass das Blank 23 dem künstlichen Gewebe entnommen wird und auf einem Halter 25 fixiert wird. Insbesondere bei der Bearbeitung von künstlichem Gewebe ist dieser Weg der bevorzugte, da bei Fixierung auf einem Halter 25 während der Bearbeitung mit der zweiten Lasereinrichtung 7, also einer Excimer-Lasereinrichtung, eine sicheres Temperaturregime gewährleistet sein sollte: Künstliches Gewebe ist diesbezüglich noch sensibler als das natürliche Gewebe einer Spender-Hornhaut.

Dabei ist in der Fig. 3a zunächst ein künstliches Gewebe dargestellt, das eine natürliche Hornhaut, mit Epithelschicht 101, Bowman-Membran 102 und Cornealem Stroma 103 quasi "nachbaut". Zudem werden die Schnitte gezeigt, die das Blank 23 ganz grundsätzlich von dem künstlichen Gewebe separieren: Der lamelläre Schnitt ("lamellar cut") 104 und der Randschnitt ("side cut") 105, der in diesem Falle nicht vom lamellären Schnitt 104 bis an in die Epithelschicht 101 geführt wird, sondern nur ein in die Stromaschicht geführt wird. Wirklich getrennt vom künstlichen Gewebe wird das Blank 23 jedoch erst durch den modifizierten Randschnitt 120.

In der Fig. 3b ist zu den bereits in der Fig. 3a genannten Bereichen, Schnitten und Markierungen noch das zu erzeugende Implantat 106 in Form der personalisierten Lamelle und daraus folgend das Ablationsvolumen 107 sowie eine Excavation 108 dargestellt: Insbesondere durch die Darstellung der Excavation 108 wird deutlich, dass der modifizierten Randschnitt 120 so geplant ist, dass die Excavation 108 im durch die Schnitte vom künstlichen Gewebe separierten Blank 23 vollständig umfasst ist. Sichtbar in der Fig. 3b sind zudem wiederum die beiden äußeren Begrenzungen der Korrekturzone 109, 110, sowie die obere äußere Begrenzung des Implantats 111, die den Schnittpunkt von Randschnitt 105 und finaler Implantat-Oberseite darstellt.

In der Fig. 3c ist das Blank 23 zunächst aus dem künstlichen Gewebe entnommen und auf einem Haller 25 fixiert worden. Neben den in den Fig. 3a und 3b genannten Bereichen, Schnitten und Markierungen ist hier zudem die äußere Begrenzung der Übergangszone 112, 113 dargestellt.

Die Fig. 3d zeigt neben den bereits in den Fig. 3a bis 3c genannten Bereichen, Schnitten und Markierungen wiederum Kalibriermarken 114. Auch hier kann mit Hilfe der Kalibriermarken 114 die Form 115 der Excavation 108 beeinflusst werden.

Die Fig. 3e zeigt neben den bereits erwähnten Kalibriermarken 114 weitere Kalibriermarken: Kalibriermarken über dem Implantat in dessen Randbereich 116 und Kalibriermarken neben dem Implantat 106, sowie gestapelte Kalibriermarken 117, 118 - dies alle in mehr oder weniger gleicher Weise wie bei Bearbeitung des Blanks 23 zu einer patientenspezifischen Lamelle 24 in der Spender-Hornhaut. Allerdings kann nun in dieser Variante ab dem Moment der Fixierung des Blanks 23 auf dem Halter 25 ein Temperaturregime bei der Bearbeitung des Blanks 23 mit der zweiten Lasereinrichtung 7, der Excimer-Lasereinrichtung, eingeführt und sehr präzise gehalten werden.

In der Fig. 3f ist zudem noch ein Randschnitt 119 mit der ersten 3 bzw. weiteren 6 Lasereinrichtung, der Femtosekunden-Lasereinrichtung, geplant, der damit die Höhe des Implantats 106 in dessen Randzone, insbesondere der patientenspezifische Lamelle 24 als Endprodukt der Planung und Bearbeitung des Blanks 23, sicher festlegt.

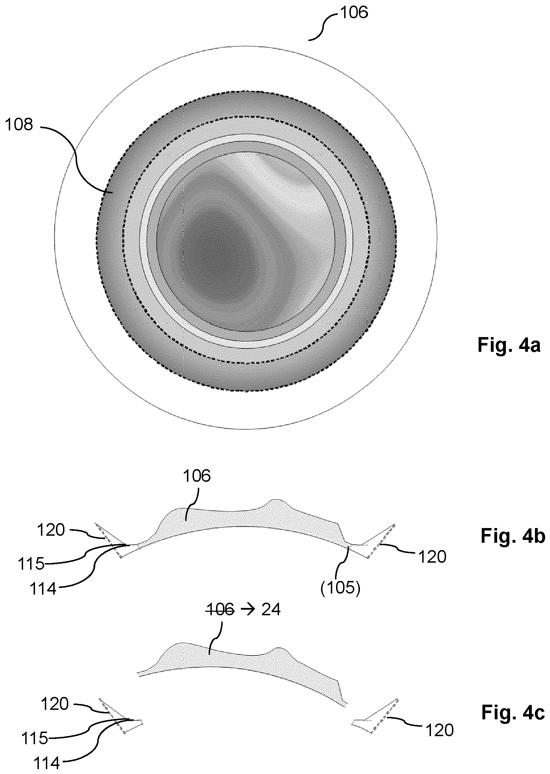

Die Fig. 4a bis 4c zeigen schlussendlich ein Implantat 106 nach Bearbeitung mit den Lasereinrichtungen 3, 7, 6 des erfindungsgemäßen Behandlungssystems 1 und vor seiner Einführung in eine Schnittfläche 21 der Hornhaut eines Empfänger-Auges.

In der Fig. 4a ist ein solches Implantat 106, dessen Blank 23 auf einem Halter 25 mit der zweiten Lasereinrichtung 7, der Excminer-Lasereinrichtung, bearbeitet wurde, in einer Draufsicht dargestellt, die Fig. 4b und 4c zeigen dasselbe Implantat 106 in einer Seitenansicht. Durch die Excavation wurde der tatsächliche Rand der zu implantierenden Lamelle 24 präzise herausgearbeitet. Er ist aber bis nach seiner Bearbeitung mit der zweiten Lasereinrichtung 7 geschützt. Die Trennstelle, um diesen erweiterten "Schutzrand" schließlich abzutrennen, wurde mit dem Randschnitt ("side cut") des Blanks 23 schon zu Beginn des gesamten Herstellungsprozesses der patientenspezifischen Lamelle 24 angelegt. Diese patientenspezifische Lamelle 24 bleibt dann nach der tatsächlichen Abtrennung des "Schutzrandes", wie in der Fig. 4c gezeigt, übrig und kann in die vorbereitete Schnittfläche 21 bzw. Struktur in der Hornhaut 20 des Empfängerauges eingeführt werden.

Die vorstehend genannten und in verschiedenen Ausführungsbeispielen erläuterten Merkmale der Erfindung sind dabei nicht nur in den beispielhaft angegebenen Kombinationen, sondern auch in anderen Kombinationen oder allein einsetzbar, ohne den Rahmen der vorliegenden Erfindung zu verlassen.

## Patentansprüche

1. Planungseinrichtung (2) zum Erzeugen von Steuerdaten für ein Behandlungssystem (1) zur refraktiven Chirurgie, insbesondere zur Keratoplastie, das eine erste Lasereinrichtung (3) und mindestens eine Charakterisierungseinrichtung (4) umfasst,
- wobei mittels der Steuerdaten die erste Lasereinrichtung (3), vorzugsweise eine Femtosekunden-Lasereinrichtung, zur Erzeugung mindestens einer Schnittfläche (21) in einer Hornhaut (20) eines Auges steuerbar ist, wobei die Planungseinrichtung (2) aufweist:
- eine Schnittstelle (5) zum Zuführen von ersten Messdaten über Parameter der Hornhaut (20) der Charakterisierungseinrichtung (4), vorzugsweise einer OCT (optische Kohärenz-Tomographie)-Einrichtung,
- eine Schnittstelle (5) zum Zuführen von zweiten Messdaten oder Modelldaten einer Lamelle (24), die in die Hornhaut (20) nach Erzeugung der Schnittfläche (21) einführbar ist,
- eine Schnittstelle (5) zum Abführen von Steuerdaten zur ersten Lasereinrichtung (3), und
- Berechnungsmittel zum Festlegen der mindestens einen Schnittfläche (21) in der Hornhaut (20) unter Verwendung der ersten Messdaten und der zweiten Messdaten oder Modelldaten, wobei die Berechnungsmittel einen Steuerdatensatz zur Ansteuerung der ersten Lasereinrichtung (3) erzeugen, wobei durch die ersten Lasereinrichtung (3) mit den Steuerdatensatz die mindestens eine Schnittfläche (21) erzeugbar ist,
- und die Planungseinrichtung weiterhin eingerichtet ist, Steuerdaten für eine zweite Lasereinrichtung (7) des Behandlungssystems (1), vorzugsweise eine Excimer-Lasereinrichtung, zu erzeugen und eine Schnittstelle (5) zum Abführen von Steuerdaten zur zweiten Lasereinrichtung (7) aufweist, wobei erste Lasereinrichtung (3), zweite Lasereinrichtung (7) und Charakterisierungseinrichtung (4) jeweils ein Gerätekoordinatensystem aufweisen, und sie mittels Registrierung der Gerätekoordinatensysteme zueinander gekoppelt bzw. koppelbar und die zugeführten zweiten Messdaten oder Modelldaten der Lamelle (24) zu diesen Gerätekoordinatensystemen registrierbar sind,
wobei
- die zweite Lasereinrichtung zur Behandlung eines Blanks (23) zur patientenspezifisch geformten Lamelle (24) eingerichtet ist, und
die zweite Lasereinrichtung (7) zur Bearbeitung des Blanks (23) zur patientenspezifisch geformten Lamelle (24) einen Halter aufweist, auf den das Blank (23) während der Bearbeitung durch die zweite Lasereinrichtung (7) fixierbar ist,
**dadurch gekennzeichnet, dass**
- die Planungseinrichtung eingerichtet ist, eine im Wesentlichen ringförmige Übergangszone am Rande der Lamelle (24) zu bestimmen, innerhalb der die Randdicke allmählich in ein patientenspezifisches Dickenprofil übergeht, und weiterhin Steuerdaten so erzeugt werden, dass keine Bearbeitung des Randes der Lamelle durch die zweite Lasereinrichtung (7) erfolgt,
- die Planungseinrichtung weiterhin eingerichtet ist, Steuerdaten für eine aktive Kühlung des Halters zu erzeugen.

2. Planungseinrichtung (2) nach Anspruch 1, die weiterhin eingerichtet ist, Steuerdaten für die erste Lasereinrichtung (3) oder eine weitere Lasereinrichtung (6), ebenfalls vorzugsweise eine Femtosekunden-Lasereinrichtung, dessen Gerätekoordinatensystem ebenfalls zu den vorgenannten Gerätekoordinatensystemen mittels Registrierung gekoppelt ist, zu generieren zur Erzeugung bzw. Vorbearbeitung des Blanks (23), wobei das Blank (23) aus einer natürlichen Spender-Hornhaut oder aus einem künstlichem Gewebe erzeugbar bzw. darin vorbearbeitbar ist durch Erzeugung einer oder mehrerer Schnittflächen (104, 105, 119, 120) in der Spender-Hornhaut oder dem künstlichen Gewebe mittels der ersten Lasereinrichtung (3) oder der weiteren Lasereinrichtung (6).

3. Planungseinrichtung (2) nach Anspruch 1 oder 2, die eingerichtet ist, Steuerdaten für ein Temperaturregime für die Einhaltung einer Temperatur kleiner einer Maximaltemperatur für die Bearbeitung des Blanks zur Lamelle (24) mit der zweiten Lasereinrichtung (7) zu erzeugen.

4. Planungseinrichtung (2) nach Anspruch 2 oder 3, die eingerichtet ist, Schnittflächen (104, 105, 119, 120) derart in der Spender-Hornhaut oder dem künstlichen Gewebe festzulegen, Steuerdaten zu erzeugen und diese an die erste Lasereinrichtung (3) oder die weitere Lasereinrichtung (6) zu übermitteln, mit denen das Blank (23) erzeugbar ist, das durch eine im Zentrum des Blanks (23) befindliche Korrekturzone, eine um diese herum angeordnete Übergangszone und eine um diese herum angeordnete Randzone, die zur späteren Abtrennung vor einer Einführung der Lamelle (24) in die Hornhaut des Auges vorgesehen ist, definiert ist, und dieses Blank (23) entnommen und auf dem Halter (25) fixiert werden kann zur Bearbeitung mit der zweiten Lasereinrichtung (3).

5. Planungseinrichtung (2) nach Anspruch 4, die eingerichtet ist, in der Übergangszone und/oder der Randzone die Lage von Kalibriermarken (114, 117, 118) zu definieren und Steuerdaten für die ersten Lasereinrichtung (3) oder die weiteren Lasereinrichtung (6) zu erzeugen mit denen diese Kalibriermarken (114, 117, 118) während einer Bearbeitung mit der ersten Lasereinrichtung (3) oder der weiteren Lasereinrichtung (6) einbringbar sind, wobei die Kalibriermarken (114, 117, 118) derart definiert sind, dass sie als ein- oder mehrfache Orientierung während einer Bearbeitung des Blanks (23) mittels der zweiten Lasereinrichtung (7) nutzbar sind.

6. Planungseinrichtung (2) nach Anspruch 5, die Kalibriermarken (114, 117, 118) definiert, die mehrfach übereinander und/oder zueinander versetzt und/oder in verschiedenen Höhen im durch die zweite Lasereinrichtung (7) zu bearbeitenden Blank angeordnet sind.

7. Planungseinrichtung (2) nach einem der Ansprüche 1 bis 6, die eingerichtet ist, ein Bearbeitungsprofil für die zweite Lasereinrichtung (7) derart zu definieren und die Steuerdaten zu bestimmen, dass das Profil der im Zentrum des Blanks (23) befindlichen Korrekturzone und der um diese herum angeordnete Übergangszone mittels der zweiten Lasereinrichtung (7) erzeugbar ist und in der um dies Übergangszone herum angeordneten Randzone eine Excavation (108) erzeugbar ist
- zur Entnahme einer aus der Spender-Hornhaut oder dem künstlichen Gewebe herausgearbeiteten Lamelle (24), oder
- zur Weiterbearbeitung des Blanks (23) auf einem Halter (25) derart, dass der Halter (25) durch einen Bearbeitungslaserstrahl der zweiten Lasereinrichtung (7) nicht getroffen werden kann.

8. Planungseinrichtung (2) nach einem der Ansprüche 1 bis 7, die eingerichtet ist, für die Erzeugung der Steuerdaten einen definierten Ausgangshydratationszustand des Blanks (23) bzw. der Lamelle (24) ex-vivo und die Änderung des Hydratationszustands der Lamelle (24) während bzw. nach der Implantation, bevorzugt mittels eines konstanten Expansionsfaktors, zu berücksichtigen.

9. Behandlungssystem (1) für die refraktiven Chirurgie, insbesondere für die Keratoplastie, das
- eine erste Lasereinrichtung (3) zur Erzeugung mindestens einer Schnittfläche (21) in einer Hornhaut (20) eines Auges, vorzugsweise eine Femtosekunden-Lasereinrichtung,
- eine zweite Lasereinrichtung (7) zur Bearbeitung eines Blanks (23) zur patientenspezifisch geformten Lamelle (24), vorzugsweise eine Excimer-Lasereinrichtung,
- mindestens eine Charakterisierungseinrichtung (4), vorzugsweise eine OCT-Einrichtung,
**dadurch gekennzeichnet, dass** das Behandlungssystem des weiteren
- eine Planungseinrichtung (2) nach einem der Ansprüche 1 bis 8 umfasst, und
- die zweite Lasereinrichtung (7) einen Halter (25) zur Fixierung des Blanks (23) während der Bearbeitung enthält.

10. Behandlungssystem (1) nach Anspruch 9, das eine weitere Lasereinrichtung (6) zur Erzeugung bzw. Vorbearbeitung des Blanks (23), vorzugsweise eine Femtosekunden-Lasereinrichtung, aufweist.

11. Behandlungssystem (1) nach einem der Ansprüche 9 bis 10, das eine Temperaturregelungseinrichtung aufweist, vorzugsweise derart, dass eine Temperatur des Blanks (23) so abgesenkt werden kann, dass das Blank (23) im gefrorenen Zustand bearbeitbar ist.

12. Behandlungssystem (1) nach Anspruch 11, dessen Temperaturregelungseinrichtung mindestens eine der folgenden Ausgestaltungen aufweist:
- eine aktive elektrische Kühlung mittels Peltier-Element,
- eine aktive Kühlung mittels eines eingebrachten Kühlmittels,
- eine aktive Kühlung durch einen Luftstrom,
- eine passive Kühlung durch Vorkühlung des Halters (25) mit oder ohne dem darauf fixierten Blank (25),
- eine von der Umgebung abgetrennten Kammer zur Prozessierung des Blanks (23).

13. Behandlungssystem (1) nach einem der Ansprüche 9 bis 12, das eine Einrichtung zur Überwachung der Temperatur des Blanks (23) und/oder des Halters (25) während der Bearbeitung aufweist.

## Claims

1. Planning device (2) for generating control data for a treatment system (1) for refractive surgery, in particular for keratoplasty, said treatment system comprising a first laser device (3) and at least one characterization device (4),
- wherein the first laser device (3), preferably a femtosecond laser device, is rendered controllable by means of the control data so as to create at least one cut surface (21) in a cornea (20) of an eye, the planning device (2) comprising:
- an interface (5) for supplying first measurement data regarding parameters of the cornea (20) to the characterization device (4), preferably an OCT (optical coherence tomography) device,
- an interface (5) for supplying second measurement data or model data about a lamella (24) which is insertable into the cornea (20) following the creation of the cut surface (21),
- an interface (5) for transmitting control data to the first laser device (3), and
- calculation means for defining the at least one cut surface (21) in the cornea (20) using the first measurement data and the second measurement data or model data, the calculation means generating a control data set for controlling the first laser device (3), and the at least one cut surface (21) being created by the first laser device (3) using the control data set,
- and the planning device is furthermore configured to generate control data for a second laser device (7) of the treatment system (1), preferably an excimer laser device, and comprising an interface (5) for transmitting control data to the second laser device (7), wherein the first laser device (3), the second laser device (7) and the characterization device (4) each have an equipment coordinate system and these devices are coupled or couplable with respect to one another by means of registration of the equipment coordinate systems, and the supplied second measurement data or model data of the lamella (24) are registrable with respect to these equipment coordinate systems,
wherein
- the second laser device is configured to treat a blank (23) to form a patient-specifically shaped lamella (24), and the second laser device (7) for processing the blank (23) to form a patient-specifically shaped lamella (24) comprises a holder on which the blank (23) is affixable during the processing by the second laser device (7), **characterized in that**
- the planning device is configured to determine a substantially ring-shaped transition zone at the edge of the lamella (24), within which the edge thickness gradually transitions into a patient-specific thickness profile, and furthermore control data are generated in such a way that there is no processing of the edge of the lamella by the second laser device (7),
and
- the planning device is furthermore configured to generate control data for active cooling of the holder.

2. Planning device (2) according to Claim 1, furthermore configured to generate control data for the first laser device (3) or a further laser device (6), likewise preferably a femtosecond laser device, the equipment coordinate system of which is likewise coupled to the aforementioned equipment coordinate systems by means of registration, in order to generate or pre-process the blank (23), wherein the blank (23) is able to be generated from a natural donor cornea or from artificial tissue, or the blank is pre-processable therein, by creating one or more cut surfaces (104, 105, 119, 120) in the donor cornea or the artificial tissue by means of the first laser device (3) or the further laser device (6).

3. Planning device (2) according to Claim 1 or 2, configured to generate control data for a temperature regime for maintaining a temperature below a maximum temperature for processing the blank to form the lamella (24) using the second laser device (7).

4. Planning device (2) according to Claim 2 or 3, configured to define cut surfaces (104, 105, 119, 120) in the donor cornea or in the artificial tissue in such a way as to generate control data and transmit the latter to the first laser device (3) or the further laser device (6) with which the blank (23) is being generated, the blank being defined by a correction zone situated in the centre of the blank (23), a transition zone arranged around said correction zone and an edge zone arranged around said transition zone, the edge zone being provided for the subsequent separation prior to an insertion of the lamella (24) into the cornea of the eye, and this blank (23) can be removed and affixed on the holder (25) for the purposes of processing with the second laser device (3).

5. Planning device (2) according to Claim **4,** configured to define the position of calibration marks (114, 117, 118) in the transition zone and/or the edge zone and to generate control data for the first laser device (3) or the further laser device (6), by means of which control data these calibration marks (114, 117, 118) are able to be introduced during processing with the first laser device (3) or the further laser device (6), wherein the calibration marks (114, 117, 118) are defined such that they are usable as a single or multiple orientation means during a processing of the blank (23) by means of the second laser device (7).

6. Planning device (2) according to Claim 5, which defines calibration marks (114, 117, 118) which are arranged multiple times above one another and/or offset from one another and/or at different levels in the blank to be processed by the second laser device (7).

7. Planning device (2) according to any of Claims 1 to 6, configured to define a processing profile for the second laser device (7) and determine the control data in such a way that the profile of the correction zone situated in the centre of the blank (23) and of the transition zone arranged around said correction zone is producible by means of the second laser device (7) and an excavation (108) is producible in the edge zone arranged around this transition zone,
- for the removal of a lamella (24) carved out of the donor cornea or the artificial tissue, or
- for further processing of the blank (23) on a holder (25), in such a way that the holder (25) cannot be hit by a processing laser beam of the second laser device (7).

8. Planning device (2) according to any of Claims 1 to 7, configured to take account of a defined initial hydration state of the blank (23) or of the lamella (24) ex vivo, and the change in the hydration state of the lamella (24) during or after the implantation, preferably by means of a constant expansion factor, to generate the control data.

9. Treatment system (1) for refractive surgery, in particular for keratoplasty, comprising
- a first laser device (3) for creating at least one cut surface (21) in a cornea (20) of an eye, preferably a femtosecond laser device,
- a second laser device (7) for processing a blank (23) to form a patient-specifically shaped lamella (24), preferably an excimer laser device,
- at least one characterization device (4), preferably an OCT device,
**characterized in that** the treatment system further
- comprises a planning device (2) according to any of Claims 1 to 8, and
- the second laser device (7) contains a holder (25) for affixing the blank (23) during the processing.

10. Treatment system (1) according to Claim 9, comprising a further laser device (6) for generating or pre-processing the blank (23), preferably a femtosecond laser device.

11. Treatment system (1) according to any of Claims 9 and 10, comprising a temperature control device, preferably such that the temperature of the blank (23) can be lowered in such a way that the blank (23) is able to be generated in the frozen state.

12. Treatment system (1) according to Claim 11, the temperature control device of which comprises at least one of the following configurations:
- active electrical cooling by means of a Peltier element,
- active cooling by means of an introduced coolant,
- active cooling by an air flow,
- passive cooling by pre-cooling the holder (25) with or without the blank (25) affixed thereon,
- a chamber, separated from the surroundings, for processing the blank (23).

13. Treatment system (1) according to any of Claims 9 to 12, comprising a device for monitoring the temperature of the blank (23) and/or of the holder (25) during the processing.

## Revendications

1. Dispositif de planification (2) pour générer des données de commande destinées à un système de traitement (1) pour la chirurgie réfractive, en particulier pour la kératoplastie, qui comprend un premier dispositif laser (3) et au moins un dispositif de caractérisation (4),
- dans lequel, au moyen des données de commande, le premier dispositif laser (3), de préférence un dispositif laser femtoseconde, peut être commandé pour générer au moins une surface de coupe (21) dans une cornée (20) d'un œil, le dispositif de planification (2) comprenant :
- une interface (5) destinée à délivrer des premières données de mesure concernant des paramètres de la cornée (20) du dispositif de caractérisation (4), de préférence un dispositif OCT (tomographie à cohérence optique),
- une interface (5) destinée à délivrer des secondes données de mesure ou des données de modèle d'une lamelle (24) qui peut être introduite dans la cornée (20) après génération de la surface de coupe (21),
- une interface (5) destinée à envoyer des données de commande au premier dispositif laser (3), et
- des moyens de calcul destinés à définir ladite au moins une surface de coupe (21) dans la cornée (20) à l'aide des premières données de mesure et des secondes données de mesure ou des données de modèle, les moyens de calcul générant un jeu de données de commande permettant de commander le premier dispositif laser (3), ladite au moins une surface de coupe (21) pouvant être générée par le premier dispositif laser (3) au moyen du jeu de données de commande,
- et le dispositif de planification est en outre conçu pour générer des données de commande destinées à un second dispositif laser (7) du système de traitement (1), de préférence un dispositif laser à excimère, et comporte une interface (5) destinée à transférer des données de commande au second dispositif laser (7), le premier dispositif laser (3), le second dispositif laser (7) et le dispositif de caractérisation (4) présentant chacun un système de coordonnées d'appareil, et étant couplés ou pouvant être couplés les uns aux autres par un calage des systèmes de coordonnées d'appareil, et les secondes données de mesure ou les données de modèle de la lamelle (24) délivrées pouvant être calées par rapport à ces systèmes de coordonnées d'appareil,
dans lequel
- le second dispositif laser est conçu pour le traitement d'une ébauche (23) en une lamelle (24) ayant une forme spécifique au patient, et le second dispositif laser (7) présente, pour le traitement de l'ébauche (23) en une lamelle (24) ayant une forme spécifique au patient, un support sur lequel l'ébauche (23) peut être fixée pendant le traitement par le second dispositif laser (7), **caractérisé en ce que**
- le dispositif de planification est conçu pour déterminer une zone de transition sensiblement annulaire à la périphérie de la lamelle (24), à l'intérieur de laquelle l'épaisseur de la périphérie se transforme progressivement en un profil d'épaisseur spécifique au patient, et des données de commande sont en outre générées de telle sorte qu'aucun traitement de la périphérie de la lamelle ne soit effectué par le second dispositif laser (7),
et
- le dispositif de planification est en outre conçu pour générer des données de commande pour un refroidissement actif du support.

2. Dispositif de planification (2) selon la revendication 1, lequel est en outre conçu pour générer des données de commande destinées au premier dispositif laser (3) ou à un autre dispositif laser (6), également de préférence un dispositif laser femtoseconde, dont le système de coordonnées d'appareil est également couplé aux systèmes de coordonnées d'appareil précités au moyen d'un calage, pour générer ou pré-traiter l'ébauche (23), l'ébauche (23) pouvant être générée à partir d'une cornée naturelle de donneur ou d'un tissu artificiel ou pouvant y être pré-traitée par génération d'une ou de plusieurs surfaces de coupe (104, 105, 119, 120) dans la cornée de donneur ou le tissu artificiel au moyen du premier dispositif laser (3) ou de l'autre dispositif laser (6).

3. Dispositif de planification (2) selon la revendication 1 ou 2, qui est conçu pour générer des données de commande destinées à un régime de température permettant de maintenir une température inférieure à une température maximale pour le traitement de l'ébauche en lamelle (24) au moyen du second dispositif laser (7).

4. Dispositif de planification (2) selon la revendication 2 ou 3, qui est conçu pour définir des surfaces de coupe (104, 105, 119, 120) dans la cornée du donneur ou dans le tissu artificiel afin de générer des données de commande et à les transmettre au premier dispositif laser (3) ou à l'autre dispositif laser (6), au moyen desquelles l'ébauche (23) peut être générée, laquelle est définie par une zone de correction se trouvant au centre de l'ébauche (23), une zone de transition disposée autour de celle-ci et une zone périphérique disposée autour de celle-ci, laquelle est destinée à être ultérieurement séparée avant l'introduction de la lamelle (24) dans la cornée de l'œil, ladite ébauche (23) pouvant être extraite et fixée sur le support (25) pour être traitée au moyen du second dispositif laser (3).

5. Dispositif de planification (2) selon la revendication 4, lequel est conçu pour définir dans la zone de transition et/ou la zone périphérique la position de marques d'étalonnage (114, 117, 118) et pour générer des données de commande destinées au premier dispositif laser (3) ou à l'autre dispositif laser (6), au moyen desquels ces marques d'étalonnage (114, 117, 118) peuvent être introduites pendant un traitement au moyen du premier dispositif laser (3) ou de l'autre dispositif laser (6), les marques d'étalonnage (114, 117, 118) étant définies de telle sorte qu'elles peuvent être utilisées comme orientation unique ou multiple pendant un traitement de l'ébauche (23) au moyen du second dispositif laser (7).

6. Dispositif de planification (2) selon la revendication 5, lequel définit des marques d'étalonnage (114, 117, 118) disposées plusieurs fois les unes au-dessus des autres et/ou décalées les unes par rapport aux autres et/ou à différentes hauteurs dans l'ébauche à être traitée par le second dispositif laser (7).

7. Dispositif de planification (2) selon l'une quelconque des revendications 1 à 6, lequel est conçu pour définir un profil de traitement pour le second dispositif laser (7) et pour déterminer les données de commande de telle sorte que le profil de la zone de correction se trouvant au centre de l'ébauche (23) et de la zone de transition disposée autour de celle-ci puisse être généré au moyen du second dispositif laser (7) et qu'une excavation (108) puisse être réalisée dans la zone périphérique disposée autour de cette zone de transition
- pour extraire une lamelle (24) générée à partir de la cornée du donneur ou du tissu artificiel, ou
- pour poursuivre le traitement de l'ébauche (23) sur un support (25) de telle sorte que le support (25) ne puisse pas être atteint par un faisceau laser de traitement du second dispositif laser (7).

8. Dispositif de planification (2) selon l'une quelconque des revendications 1 à 7, lequel est conçu pour prendre en compte, en vue de la génération des données de commande, un état d'hydratation initial défini de l'ébauche (23) ou de la lamelle (24) ex-vivo et pour modifier l'état d'hydratation de la lamelle (24) pendant ou après l'implantation, de préférence au moyen d'un facteur de dilatation constant.

9. Système de traitement (1) pour la chirurgie réfractive, en particulier pour la kératoplastie, lequel comprend
- un premier dispositif laser (3) destiné à générer au moins une surface de coupe (21) dans une cornée (20) d'un œil, de préférence un dispositif laser femtoseconde,
- un second dispositif laser (7) destiné à traiter une ébauche (23) en une lamelle (24) ayant une forme spécifique au patient, de préférence un dispositif laser à excimère,
- au moins un dispositif de caractérisation (4), de préférence un dispositif OCT,
**caractérisé en ce qu'**en outre, le système de traitement
- comprend un dispositif de planification (2) selon l'une quelconque des revendications 1 à 8, et
- **en ce que** le second dispositif laser (7) contient un support (25) permettant de fixer l'ébauche (23) pendant le traitement.

10. Système de traitement (1) selon la revendication 9, qui comprend un autre dispositif laser (6) destiné à générer ou à pré-traiter l'ébauche (23), de préférence un dispositif laser femtoseconde.

11. Système de traitement (1) selon l'une quelconque des revendications 9 à 10, lequel comprend un dispositif de régulation de la température, de préférence de telle sorte qu'une température de l'ébauche (23) puisse être abaissée afin que l'ébauche (23) puisse être traitée à l'état congelé.

12. Système de traitement (1) selon la revendication 11, dont le dispositif de régulation de la température présente au moins l'une des configurations suivantes :
- un refroidissement électrique actif au moyen d'un élément Peltier,
- un refroidissement actif au moyen d'un fluide de refroidissement introduit,
- un refroidissement actif par un courant d'air,
- un refroidissement passif par pré-refroidissement du support (25) avec ou sans l'ébauche (25) fixée sur celui-ci,
- une chambre séparée de l'environnement pour le traitement de l'ébauche (23).

13. Système de traitement (1) selon l'une quelconque des revendications 9 à 12, lequel comprend un dispositif de surveillance de la température de l'ébauche (23) et/ou du support (25) pendant le traitement.
